# EUROPEAN PATENT SPECIFICATION

(11) **EP 2 495 336 B1**
(45) Date of publication and mention of the grant of the patent: **16.04.2014**
(21) Application number: 11001840.5
(22) Date of filing: 04.03.2011
(51) Int. Cl.: C12Q 1/68

(54) **A new type of universal probes for the detection of genomic variants**
Neuer Typ von universellen Sonden zur Detektion von Genomvarianten
Nouveau type de sondes universelles pour la détection des variantes génomiques

(43) Date of publication of application: 05.09.2012
(73) Proprietor: Roche Diagnostics GmbH, 68305 Mannheim (DE); F.Hoffmann-La Roche AG, 4070 Basel (CH)
(72) Inventor: Beck, Reinhard, 82377 Penzberg (DE); Bergmann, Frank, 82393 Iffeldorf (DE); Härteis, Rita, 82431 Ried bei Kochel am See (DE); Heindl, Dieter, 82369 Pähl (DE); Mauritz, Ralf, 82377 Penzberg (DE)
(74) Representative: Bösl, Raphael Konrad

(56) References cited:
- WO-A2-2004/113563
- SIMEONOV ANTON ET AL: "Single nucleotide polymorphism genotyping using short, fluorescently labeled locked nucleic acid (LNA) probes and fluorescence polarization detection.", NUCLEIC ACIDS RESEARCH 1 SEP 2002 LNKD- PUBMED:12202779, vol. 30, no. 17, 1 September 2002 (2002-09-01), page E91, XP2313951, ISSN: 1362-4962
- YOU YONG ET AL: "Design of LNA probes that improve mismatch discrimination", NUCLEIC ACIDS RESEARCH, vol. 34, no. 8, 2006, XP002498572, ISSN: 0305-1048

### SEQUENCE LISTING

<110> Roche Diagnostics GmbH
   F. Hoffmann-La Roche AG
<120> A new type of universal probes for the detection of genomic variants
<130> R67345EP
<160> 4
<170> PatentIn version 3.5
<210> 1
   <211> 18
   <212> DNA
   <213> Artificial sequence
<220>
   <223> Primer forward sequence
<400> 1
   gacggaccag agcgaaag 18
<210> 2
   <211> 19
   <212> DNA
   <213> Artificial sequence
<220>
   <223> Primer reverse sequence
<400> 2
   cgtcttcgaa cctccgact 19
<210> 3
   <211> 21
   <212> DNA
   <213> Artificial sequence
<220>
   <223> Primer forward sequence
<400> 3
   cccaaacctg taaaaccagt g 21
<210> 4
   <211> 21
   <212> DNA
   <213> Artificial sequence
<220>
   <223> Primer reverse sequence
<400> 4
   ttgtgaatag gtgccagtga a 21

## Description

The present invention relates to a composition comprising a first set of probes and a second set of probes, composed of one or more DNA nucleotide(s) and five or more LNA (locked nucleic acid) nucleotides (or LNA analogues like e.g. ENA (2'-O,4'-C-ethylene-bridges nucleic acid) or 2'-amino-LNA derivatives), wherein the probes differ in one, two or three LNA random position(s) and the base at a discriminating position. Further, the present invention relates to a method of detecting genomic variants by means of the aforementioned probes. Moreover, the invention relates to a library of a plurality of probes.

It is widely known that genetic polymorphisms play a crucial role for the health and the predisposition of various diseases in nearly all organisms. Genetic polymorphisms and mutations may have a great impact on the metabolism of the affected organism when a gene is affected or remain unnoticed (silent mutation) if a non-coding part of the genome is affected. In general, genetic polymorphisms are caused by mutation in a gene. The mutation may be frameshift mutation, a deletion of a gene or a part of the gene, the repetition of a gene, the insertion of a gene or a single nucleotide exchange.

The deletion of a gene or a part thereof and the alteration in the copy numbers of a gene leads to a copy number variant (CNV). CNVs can be caused by genomic rearrangements such as deletions, duplications, and translocations. CNVs are found in the connection of different types of cancer, such as, e.g., non-small cell lung cancer, but also in the connection autism and schizophrenia.

A single nucleotide exchange leads to a single nucleotide polymorphism (SNP). Numerous diseases are associated with SNP, such as, e.g., sickle-cell anaemia, hypercoagulability disorder associated with the variant Factor V Leiden, asthma predisposition, and cancer (e.g., oncogenes).

Therefore, in particular, single nucleotide polymorphisms (SNPs) and copy number variants (CNVs) play a crucial role.

Consequently, analytic tools for detecting a mutation, in particular a CNV or an SNP, in biological samples have a great impact in today's research, in more particular a CNV or an SNP, in patient samples have a great impact in today's medicine. In particular, in the view of a population that is more and more aged and wherein health care and health protection has an increasing importance, the detection of a genetic predisposition or a genetic disease becomes even more important. Further, the still rather novel field of personalized medicine essentially bases on the detection of genetic polymorphisms.

Accordingly, several methods for detecting single nucleotide polymorphisms (SNPs) and copy number variants (CNVs) have been developed in the last years. For instance, genetic polymorphisms may be detected by methods such as, e.g., sequencing of the gene of interest, single-base extension (SBE), deoxyribonucleic acid (DNA) microarrays, such as, e.g., an SNP array or an Affymetrix™ chip, PCR based on TaqMan® probes, array comparative genomic hybridization, or comparative *in situ* hybridization.

WO2004/1135 discloses libraries of short LNA probes where all sequence possibilities are covered. Probes with defined sequences are taken are interalia used in polymorphism detection assays. WO 2004/113563 does not provide compositions with probes comprising randomized LNA nucleotides as specified in the claims.

However, for all of the above methods, the synthesis of highly specific probes is required. Thus, for each potential locus of a polymorphism or a mutation, at least two highly specific probes representing two different genotypes have to be synthesized and compared with another. Consequently, for each allele in question, a probe has to be individually synthesized. The analysis of numerous potential polymorphisms loci in one experiment is therefore highly time-consuming, laborious and costly. For some of the above methods, each probe has further to be labeled. This labeling procedure is again time-consuming, laborious and costly. Other methods, such as the array-based methods, provide thousands of different variants, but are hard to analyze and do not give quantitative results. Moreover, these methods fail to detect unknown mutations. In order to achieve high specificity, the probes must have a certain minimal length. Consequently, probes with a high specificity are comparably long and are therefore difficult to synthesize. Moreover, such long probes mostly fail to detect single nucleotide polymorphisms (SNPs) as the rational differences of a fulmatch and a single mismatch are too small.

On the other hand, there are methods for the quantification of expression levels. For instance, a polymerase chain reaction (PCR) may be performed on a real-time PCR cycler such as, e.g., a LightCycler®. The PCR can also be a reverse transcriptase PCR. Further, the PCR performed on a real-time PCR cycler can be combined with intercalating DNA dyes, such as, e.g., SYBR Green I, or with a labeled probe, such as a specific or semi-specific probe. Moreover, such semi-specific probe may be, e.g., an universal probe library™ (UPL) probe. An UPL consists of probes comprising LNA (locked nucleic acid) nucleotides instead of DNA nucleotides. The probes are designed in a way to bind to numerous targets. Some of the probes bind to more than 7,000 transcripts in a single transcriptome. However, the methods based on said probes regularly fail to detect or quantify defined mutations. Therefore, the use of an semi-specific probe, such as, e.g., an UPL probe, fails to bind to a defined locus specifically.

Therefore, there is still an unmet need for a probe that consists of a short nucleic acid strand that can, on the one hand, be used universally for the detection of various target sequences and that is, on the other hand, allele specific and enables the detection of a specific mutation, in particular of a single nucleotide polymorphism (SNP).

In the context of the present invention, we provide a set of probes comprising DNA and LNA nucleotides. Preferably, at the 5' end, the nucleobases are determined, whereas at the 3' end, there are one or more, preferably two or three, random nucleotides (wobble positions). This probe design enables that a manageable number of probes is sufficient to be applicable to a multitude of problems.

Surprisingly, when using the set of probes as defined herein, the probes binding to specific targets was sufficient to discriminate different alleles of specific mutations, in particular single nucleotide polymorphisms (SNPs). Concomitantly, each set of probes can surprisingly be used for a plenty of different target genes to detect SNPs.

A first aspect of the present invention relates to a composition comprising a first set of probes and a second set of probes, each of the probes having eight nucleotides, wherein the eight nucleotides are composed of one to three DNA nucleotides and five to seven LNA (locked nucleic acid) nucleotides, wherein all probes of the first and the second set of probes have identical nucleotide sequences with the exception of
(i) the base(s) at one, two or three LNA random position(s); and
(ii) the base at a discriminating position,
wherein the one, two or three LNA random position(s) and the discriminating position are located at identical positions in all probes of the first and the second set;
wherein at each LNA random position the base is independently selected from adenine, cytosine, guanine and thymine and any possible sequence resulting from the base variation(s) at the one, two or three LNA random position(s) is represented by at least one probe in each set of probes; and
wherein the base at the discriminating position is identical within each set of probes, but differs between the first and the second set of probes.

In the context of the present invention, the term "composition" may be understood in the broadest sense as any mixture that comprises a first set of probes and a second set of probes. The composition may further comprise a solvent suitable for the probes of the present invention. This solvent may be, e.g., water, an aqueous buffer (comprising, e.g., TAPS (3-{[tris(hydroxymethyl)methyl]amino}propanesulfonic acid), Bicine (N,N-bis(2-hydroxyethyl)glycine). Tris (tris(hydroxymethyl)methylamine), Tricine (tris(hydroxymethyl)methylglycine), HEPES (2-hydroxyethyl-1-piperazineethanesulfonic acid), TES (2-{[tris(hydroxymethyl)methyl]amino}ethanesulfonic acid, MOPS (3-(N-morpholino)propanesulfonic acid), PIPES (piperazine-N,N'-bis(2-ethanesulfonic acid)), Cacodylate (dimethylarsinic acid), SSC (saline sodium citrate), MES (2-(N-morpholino)ethanesulfonic acid), phosphate, hydrogen phosphate, dihydrogen phosphate, citrate, acetate, and/or borax), an organic solvent, (e.g. dimethyl sulphoxide (DMSO), dimethylformamide (DMF)) or a combination thereof. Further, the composition may contain inorganic and/or organic salt(s), in particular the composition may contain magnesium, sodium, potassium, calcium, chloride, phosphate, hydrogen phosphate, dihydrogen phosphate, citrate, acetate, and/or borax salts. The composition may also comprise other substances, such as, e.g. biological substances (e.g., proteins, peptides, amino acids, saccharides, lipids, etc.), synthetic polymers (e.g., polyethylene imine (PEI), hydroxypropylmethacrylamide (HPMA), polyethylene glycol (PEG)), DNA-staining fluorescence dyes (e.g., SYBR green, ethidium bromide), one or more detergents, surfactants and/or emulgators (e.g., sodium dodecylsulfate (SDS)), one or more chelators (e.g., ethylenediaminetetraacetate (EDTA)), therapeutic agents, or combinations of two or more thereof.. Alternatively, the composition may be dried or freeze-dried. Alternatively, the probes may also be immobilized on a solid support, such as an array surface and/or a bead surface.

As used in the context of the present invention, the term "nucleotide" may be understood in the broadest sense as a monomer of a deoxyribonucleic acid (DNA) or a locked nucleic acid (LNA) strand (or an LNA analogue). As will be understood by a person skilled in the art, each nucleotide of the probe comprises one nucleobase. The terms "base" and "nucleobase" as used herein may be understood interchangeably in the broadest sense as understood by the person skilled in the art. The nucleobase may be e.g., adenine (A), thymine (T), cytosine (C), guanine (G), uracil (U), methyl cytosine (mC).

The nucleotides of the present invention are conjugated by ester formation of the 3' and the 5' hydroxyl groups of the ribose, deoxyribose and/or ribose derivatives (e.g., locked ribose) with phosphate anions as widely known in the art. There may be no other covalently bound molecular moieties linking the nucleotides of the strand with another than nucleotides and phosphate anion moieties. In particular, there may be no linkers or spacers located between the nucleotides. In the context of the present invention, the length of the plain nucleic acid strand is eight nucleotides in length. It will be understood that this length refers to the length of the nucleic acid strand, but may not exclude that other molecular moieties (such as, e.g., one or more fluorophore(s), one or more quencher(s), one or more binding moiety/moieties or the like) may be added to the probe, in particular may be added at the end of the probe. The term "end" of the probe may be understood as the "3' end" or the "5' end" of the probe. Herein, the term "end" and the term "terminus" may be understood interchangeably. In particular, a molecular moiety may be conjugated to the 3' and/or the 5' hydroxyl group of the probe. The person skilled in the art will notice that the terms "5' end" as used herein may refer to the 5' end of the nucleotide strand, but may not exclude that at the 5' end another molecular moiety (such as, e.g., a fluorophore, a quencher, a binding moiety or the like) is added to the 5' end of the probe. The person skilled in the art will notice that the terms "3' end" as used herein may refer to the 3' end of the nucleotide strand, but may not exclude that at the 3' end another molecular moiety (such as, e.g., a fluorophore, a quencher, a binding moiety or the like) is added to the 3' end of the probe.

As used in the context of the present invention, the terms "locked nucleic acid" and "LNA", respectively, may be understood in the broadest sense as a nucleotide, wherein the ribose ring is "locked" with an extra bridge connecting the 2'-oxygen atom with the 4'-carbon atom of the nucleotide (e.g., a methylene bridge) (WO 99/14226). Therefore, LNA may be understood as modified, inaccessible RNA, wherein the bridge "locks" the ribose in the 3' endo confirmation. LNA nucleotides as well as LNA oligomers are commercially available. The locked ribose confirmation is known to enhance base stacking and backbone pre-organization, significantly increasing the hybridization properties with a DNA or RNA target strand and, therefore, increasing the binding strength per base pair (increased thermal stability/melting temperature). LNAs have been used for DNA microarrays, as FISH probes and as real-time PCR probes. LNAs are widely resistant to endo- and exonuclease activity. Alternative LNA nucleotides are e.g. ENA (2'-O,4'-C-ethylene-bridges nucleic acid) or 2'-amino-LNA derivatives (see K. Morita et al., Bioorg. Med. Chem. Lett. 2002, 12, 73-76 and S.K. Singh et al., J. Org. Chem. 1998, 63, 10035) or 5' Methyl LNA derivatives (see WO 2010077578). Further alternatives are Blocked Nucleic Acids BNA (see US Patent No 7,427,672 and US Patent No 7,217,805) or bicyclic cyclohexitol nucleic acid (see WO 2009100320). LNA may be combined with other nucleotides, such as, e.g., DNA nucleotides. Also these oligomers are commercially available. As used herein, the nucleic acid strand as used in the context of the present invention is a molecule comprising n DNA nucleotides (n = 1, 2, or 3) and 8-n locked nucleic acid (LNA) nucleotides.

As used herein, the terms "first set of probes" and "second set of probes" refer to two sets of probes, wherein both sets of probes have identical nucleotide sequences except for the base(s) at the LNA random position(s); and the base at a discriminating position.

In the context of the present invention, the term "LNA random position" refers to a position in the nucleotide sequence, wherein the nucleobase is any nucleotide of the group consisting of adenine (A), thymine (T), cytosine (C) or guanine (G). Alternatively, the nucleobase may also be uracil (U) or methyl cytosine (mC) or another nucleobase that can form a base pair with a complementary nucleobase. In the context of the present invention, the terms "random position" and "wobble position" may be understood interchangeably. The nucleobase is independently selected from the nucleobases A, T, C and G. In the context of the present invention, there are one, two, or three LNA random position(s) in each probe of the first and the second set of probes. It will be understood that a set of probes of the present may contain 4" probes of different sequences, wherein n refers to the number of LNA random position(s). Thus, when there is one random position, as exemplified below, n = and 4¹ = 4, the set of probes would contain 4 different probes. Accordingly, when there are two LNA random positions in each probe, n = 2 and 4² = 16, the set of probes would contain 16 different probes and when there are three LNA random positions in each probe, n = 3 and 4³ = 64, the set of probes would contain 64 different probes. The one, two, or three LNA random position(s) are located at the identical position in all probes of the first and the second set.

In order to exemplify the probe design in a non-limiting example, a composition comprising a first set of probes and a second set of probes having one LNA random position may, for instance, have the following sequences. The first set of probes may be a mixture of the following probes:
5'-¹C_{(DNA)}- ²G(_{LNA}) - ³T_{(LNA)} - **⁴A_{(LNA})** - ⁵A_{(LNA)} - ⁶G_{(LNA)} - ⁷T_{(LNA)} - **⁸A_{(LNA)}-3',**
5'-¹C_{(DNA)} - ²G_{(LNA)} - ³T_{(LNA)} - **⁴A_{(LNA)} -** ⁵A_{(LNA)} - ⁶G_{(LNA)} - ⁷T_{(LNA)} - **⁸T_{(LNA)}**-3',
5'-¹C_{(DNA)} - ²G_{(LNA)} - ³T_{(LNA)} - **⁴A_{(LNA)} -** ⁵A_{(LNA)} - ⁶G_{(LNA)} - ⁷T_{(LNA)} - **⁸C_{(LNA)}**-3', and
5'-¹C_{(DNA)} - ²G_{(LNA)} - ³T_{(LNA)} - **⁴A_{(LNA)} -** ⁵A_{(LNA)} - ⁶G_{(LNA)} - ⁷T_{(LNA)} - **⁸G_{(LNA)}**-3'.

The corresponding second set of probes may be a mixture of the following probes:
5'-¹C_{(DNA)}- ²G_{(LNA)} - ³T_{(LNA)} - **⁴C_{(LNA)}** - ⁵A_{(LNA)} - ⁶G_{(LNA)} - ⁷T_{(LNA)} - **⁸A_{(LNA)}**-3',
5'-¹C_{(DNA)} - ²G_{(LNA)} - ³T_{(LNA)} - **⁴C_{(LNA)}** - ⁵A_{(LNA)} - ⁶G_{(LNA)} - ⁷T_{(LNA)} - **⁸T_{(LNA)}**-3',
5'-¹C_{(DNA)} - ²G_{(LNA)} - ³T_{(LNA)} - **⁴C_{(LNA)}** - ⁵A_{(LNA)} - ⁶G_{(LNA)} - ⁷T_{(LNA)} - **⁸C_{(LNA)}**-3', and
5'-¹C_{(DNA)} - ²G_{(LNA)} - ³T_{(LNA)} - **⁴C_{(LNA)}** - ⁵A_{(LNA)} - ⁶G_{(**LNA)**} - ⁷T_{(LNA)} - **⁸C_{(LNA)}**-3', and 5'-¹C_{(DNA)} - ²G_{(LNA)} - ³T_{(LNA)} - **⁴C_{(LNA)}** - ⁵A_{(LNA)} - 6G_{(LNA)} - ⁷T_{(LNA)} - **⁸G(_{LNA)}**-3'.

Herein, the superscript digits 1-8 characterize the nucleotide position in the probe from the 5' end. The subscript three-letter codes in parentheses show whether the nucleotide is a deoxyribonucleic acid (DNA) nucleotide or a locked nucleic acid (LNA) nucleotide.

As can be seen from the above example, the probes are eight nucleotides in length. The nucleotide in position 1 from the 5' end is a DNA nucleotide. The nucleotides in positions 2-8 from the 5' end are LNA nucleotides. The LNA random position is located in position 8 from the 5' end. Therefore, each set of probes contains four different probes. In the above example, the discriminating position is located at position 4 from the 5' end of the probes. All four probes of the first set of probes bear an adenine (A) nucleobase at position 4 from the 5' end, thus, at the discriminating position, whereas all four probes of the second set of probes bear a cytosine (C) nucleobase at the discriminating position.

It will be understood that the above example is intended to explain the probe design of a typical probe of the present invention, but is not intended to limit the scope of the present invention.

As used herein, the term "discriminating position" refers to a position in the probe, where a nucleotide differs in the two sets of probes. The nucleobase at the discriminating position is identical within each set of probes, but differs between the first and the second set of probes. An adenine moiety may be replaced by thymine, cytosine or guanine. A thymine moiety may be replaced by guanine, cytosine or adenine. A cytosine moiety may be replaced by thymine, guanine or adenine. A guanine moiety may be replaced by thymine, cytosine or adenine. In the context of the present invention, the discriminating position is located at the identical position in all probes of the first and the second set.

The term "identical position" as used herein refers to the position in the probe sequence. In the context of the present invention, the term "position" refers to the nucleotide position from the 5' end as regularly used for nucleic acid sequences. The term "identical sequence" refers to two sequences that have each the same type of nucleotide, thus, also the same type of nucleobase, at an identical position of the probe.

The composition may comprise two, three, four, five or more different set of probes that may each detect a particular genotype of a particular locus. In the context of the present invention, the term "locus" may be understood in the broadest sense as a position in the nucleotide sequence of a gene, in particular a target gene to which the probe of the invention may bind. The locus may be also designated as the part of the target sequence in question or the target sequence. A potential mutation may be located at the locus. Alternatively, the locus does not comprise a mutation. The locus may be a gene wherein a frameshift mutation occurs, a part of a gene wherein a frameshift mutation occurs, a group of two, three, four, five or more nucleotides or a single nucleotide.

The term "genotype" may be understood in the broadest sense as the genetic makeup of an organism or a virus (i.e. the specific allele makeup of the organism or virus). As used herein, the term "organism" refers to all living beings, such as, e.g., bacteria, archaebacteria, animals, plants, fungi. Further the term organism may refer to the dead body of a being that has been a living being before. The term "virus" may include virus-like particles.

The term "mutation" as used herein, may be understood in the broadest sense as an alteration in the nucleotide sequence. A mutation may occur in an encoding section of the genome or may occur in a non-encoding section of the genome. A mutation occurring in an encoding section of the genome may result in an altered amino acid sequence of a polypeptide encoded by said gene when the gene is expressed. Alternatively, a mutation may be a silent mutation, wherein the amino acid sequence of the expressed polypeptide is not affected or wherein the mutation occurs in a non-encoding region of the genome. A mutation may occur naturally in a population. Alternatively, a mutation may be provoked by xenobiotics or radiation. Such xenobiotics may be a mutagenic agent as known in the art (e.g., alkylating agent (e.g., nitrogen mustards (e.g., Cyclophosphamide, Mechlorethamine or mustine (HN2), Uramustine or uracil mustard, Melphalan, Chlorambucil, Ifosfamide), nitrosoureas (e.g., Carmustine, Lomustine, Streptozocin), alkyl sulfonates (e.g., Busulfan), thiotepa and its analogues, platinum derivates (e.g., Cisplatin, Carboplatin, Nedaplatin, Oxaliplatin, Satraplatin, Triplatin tetranitrate), Procarbazine, altretamine, aflatoxine and aflatoxine and metabolic products and derivatives thereof, nitrite, aniline and metabolic products and derivatives thereof, benzene and metabolic products and derivatives thereof, polycyclic aromatics and metabolic products and derivatives thereof) to a nucleobase), nitrosamines, arsenic, asbestos, beryllium and its compounds, ethylene oxide, hexavalent chromium(VI) compounds, radon, vinyl chloride, smoking, etc.). Such radiation may be, e.g., ultra violet (UV) radiation, X-ray radiation, radioactive/nuclear radiation (e.g., alpha-, beta- or gamma-radiation) or cosmic radiation.

Herein, the term "gene" may be understood in the broadest sense as known in the art as a unit of the nucleotide sequence of a genome as known in the art. The genome is the entity of the genes of an organism.

A mutation may be, e.g., a frameshift mutation, a deletion of a gene or a part of the gene, the repetition of a gene, the insertion of a gene or a single nucleotide exchange. Additionally, also the conjugation of molecular moieties to one or more nucleobases may be understood as a mutation, in particular when said conjugation may lead to alterations in the transcription and/or translation product. Such conjugation may be any conjugation known in the art such as, e.g., methylation of a nucleobase, loss of a methylene group of a nucleobase, conjugation of an alkylating agent (e.g., nitrogen mustards (e.g., Cyclophosphamide, Mechlorethamine or mustine (HN2), Uramustine or uracil mustard, Melphalan, Chlorambucil, Ifosfamide), nitrosoureas (e.g., Carmustine, Lomustine, Streptozocin), alkyl sulfonates (e.g., Busulfan), thiotepa and its analogues, platinum derivates (e.g., Cisplatin, Carboplatin, Nedaplatin, Oxaliplatin. Satraplatin, Triplatin tetranitrate), Procarbazine, altretamine, aflatoxine and aflatoxine and metabolic products and derivatives thereof, nitrite, nitrosamine, aniline and metabolic products and derivatives thereof, benzene and metabolic products and derivatives thereof, polycyclic aromatics and metabolic products and derivatives thereof) to a nucleobase.

In the context of the present invention, the mutation is preferably a deletion of a gene or a part of the gene, the repetition of a gene, or a single nucleotide exchange. Most preferably, the mutation is a single nucleotide exchange (SNP).

A mutation may result in an altered nucleotide sequence, such as a n altered DNA sequence. Therefore, a mutation may result in different alleles of a gene. Alternatively, a mutation may result in different alleles in a non-encoding part of the genome. The existence of at least two different alleles may be understood as a polymorphism. However, a polymorphism may also occur naturally throughout the population. Therefore, the term "polymorphism" as used herein may be understood in the broadest sense as the occurrence of different genotypes of a specific gene. The term "genotype" may be understood in the broadest sense as the nucleotide sequences in a gene. The different forms of a gene occurring due to a polymorphism, thus, the polymorphic forms may also be designated as "alleles". Throughout the population, there may be two, three, four, five, six or more different alleles of a gene. As used in the context of the present invention, the term "polymorphism" is not referred to certain incidence of a mutated gene throughout the population and may also comprise a single individual that shows a specific genotype.

The polymorphism may result in different phenotypes or may be silent. Herein, the term "silent" means that, though there are different genotypes, the phenotypes are not distinguishable by the methods known in the art.

Preferably, the polymorphism leads to different phenotypes. Some phenotypes may lead to certain diseases or pathologic conditions that occur as a direct result of the altered nucleotide sequences (e.g., cancer, sickle-cell anaemia, hypercoagulability). Alternatively, different phenotypes may result in differences in the predisposition to certain diseases or pathologic conditions (e.g., cancer, autism, schizophrenia, diabetes mellitus) and/or different phenotypes may result in differences in the metabolism, such as, e.g., xenobiotic metabolizing enzyme polymorphisms in the alcohol dehydrogenase, aldehyde dehydrogenase, glutathione-S-transferase, glucoronosyl transferase or a member of the cytochrome P 450 (CYP) superfamily.

A polymorphism that results from a single nucleotide exchange may be designated as a single nucleotide polymorphism (SNP). A polymorphism that results from a deletion of a gene or a part thereof or the alteration in the copy numbers of a gene may be designated as a copy number variant (CNV).

A single discriminating position may be used to detect a point mutation on a certain locus of the target DNA. As used herein the terms "point mutation", "single base pair mutation", "single base substitution" or other expressions known by those skilled in the art may be understood interchangeably. The position on the target gene may be understood as the locus of interest.

In a preferred embodiment of the present invention, the discriminating position is at position 2, 3, 4, 5, 6 or 7 from the 5' end in each probe, preferably at position 3, 4, or 5, more preferably at position 4.

The nucleotide at position 1 from the 5' end may be an LNA nucleotide or a DNA nucleotide. In the most preferred embodiment, the nucleotide at position 1 from the 5' end is a DNA nucleotide.

Each probe of the present invention comprises from one to three DNA nucleotides and from five to seven LNAs. It may be understood by a person skilled in the art that the probe may further comprise one or more non-nucleotide moiety/moieties such as, e.g. one or more fluorophore(s), one or more quencher(s), one or more linker(s) (e.g., an alkyl linker, a PEG linker, a peptidic linker, a saccharide linker), one or more non-fluorescent dye(s) (e.g., a dinitrophenyl moiety or Malachite Green), one or more binding moiety/moieties that can bind to other molecules (e.g., a maleimide, an isothiocyanate or an active ester (e.g., succinimidyl ester, p-nitrophenylester)), and/or one or more moiety/moieties selectively binding to high-molecular weight molecules (e.g., biotin, methotrexate or glycocorticoids). A probe conjugated with biotin may be detected by using labeled strepavidine. A probe conjugated to methotrexate may be detected by using labeled dihydrofolate reductase (DHFR). A probe conjugated with a glycocorticoid may be detected by an antibody or an antibody derivative (e.g., Fab fragment, a single chain antibody, a diabody, a triabody, a tandab).

In a preferred embodiment of the present invention, each probe consists of
- one DNA and seven LNA nucleotides,
- two DNA and six LNA nucleotides, or
- three DNA and five LNA nucleotides.

It may be understood by a person skilled in the art that, herein, the term "consists of" merely refers to the nucleotide content of the probes, but does not exclude that the probe may further comprise non-nucleotide moieties such as, e.g. fluorophores, quenchers, binding molecules and linkers as specified herein.

More preferably, the one, two or three DNA nucleotides of the probe may be located near the 5' end of said probe. Even more preferably, at least one of the five 5'-terminal nucleotides is a DNA nucleotide. Even more preferably,
the probe has a DNA nucleotide in position 1 from the 5' end,
the probe has a DNA nucleotide in position 2 from the 5' end,
the probe has a DNA nucleotide in position 3 from the 5' end,
the probe has a DNA nucleotide in position 4 from the 5' end,
the probe has a DNA nucleotide in position 5 from the 5' end,
the probe has DNA nucleotides in positions 1 and 2 from the 5' end,
the probe has DNA nucleotides in positions 1 and 3 from the 5' end,
the probe has DNA nucleotides in positions 1 and 4 from the 5' end,
the probe has DNA nucleotides in positions 1 and 5 from the 5' end,
the probe has DNA nucleotides in positions 2 and 3 from the 5' end,
the probe has DNA nucleotides in positions 2 and 4 from the 5' end,
the probe has DNA nucleotides in positions 2 and 5 from the 5' end,
the probe has DNA nucleotides in positions 3 and 4 from the 5' end,
the probe has DNA nucleotides in positions 3 and 5 from the 5' end,
the probe has DNA nucleotides in positions 4 and 5 from the 5' end,
the probe has DNA nucleotides in positions 1, 2 and 3 from the 5' end,
the probe has DNA nucleotides in positions 1, 2 and 4 from the 5' end,
the probe has DNA nucleotides in positions 1, 2 and 5 from the 5' end,
the probe has DNA nucleotides in positions 1, 3 and 4 from the 5' end, the
probe has DNA nucleotides in positions 1, 3 and 5 from the 5' end,
the probe has DNA nucleotides in positions 1, 4 and 5 from the 5' end,
the probe has DNA nucleotides in positions 2, 3 and 4 from the 5' end,
the probe has DNA nucleotides in positions 2, 4 and 5 from the 5' end,
the probe has DNA nucleotides in positions 3, 4 and 5 from the 5' end,
the probe has DNA nucleotides in positions 1, 2, 3 and 4 from the 5' end,
the probe has DNA nucleotides in positions 2, 3, 4 and 5 from the 5' end,
the probe has DNA nucleotides in positions 1, 3, 4 and 5 from the 5' end,
the probe has DNA nucleotides in positions 1, 2, 4 and 5 from the 5' end,
the probe has DNA nucleotides in positions 1, 2, 3 and 5 from the 5' end, or
the probe has DNA nucleotides in positions 1, 2, 3, 4 and 5 from the 5' end.

Even more preferably, at least one of the four 5'-terminal nucleotides is a DNA nucleotide. Even more preferably,
the probe has a DNA nucleotide in position 1 from the 5' end,
the probe has a DNA nucleotide in position 2 from the 5' end,
the probe has a DNA nucleotide in position 3 from the 5' end,
the probe has a DNA nucleotide in position 4 from the 5' end,
the probe has DNA nucleotides in positions 1 and 2 from the 5' end,
the probe has DNA nucleotides in positions 1 and 3 from the 5' end,
the probe has DNA nucleotides in positions 1 and 4 from the 5' end,
the probe has DNA nucleotides in positions 2 and 3 from the 5' end,
the probe has DNA nucleotides in positions 2 and 4 from the 5' end,
the probe has DNA nucleotides in positions 3 and 4 from the 5' end,
the probe has DNA nucleotides in positions 1, 2 and 3 from the 5' end,
the probe has DNA nucleotides in positions 1, 2 and 4 from the 5' end,
the probe has DNA nucleotides in positions 1, 3 and 4 from the 5' end,
the probe has DNA nucleotides in positions 2, 3 and 4 from the 5' end, or
the probe has DNA nucleotides in positions 1, 2, 3 and 4 from the 5' end.

Even more preferably, at least one of the three 5'-terminal nucleotides is a DNA nucleotide. Even more preferably,
the probe has a DNA nucleotide in position 1 from the 5' end,
the probe has a DNA nucleotide in position 2 from the 5' end,
the probe has a DNA nucleotide in position 3 from the 5' end,
the probe has DNA nucleotides in positions 1 and 2 from the 5' end,
the probe has DNA nucleotides in positions 1 and 3 from the 5' end,
the probe has DNA nucleotides in positions 2 and 3 from the 5' end, or
the probe has DNA nucleotides in positions 1, 2 and from the 5' end.

Even more preferably, at least one of the two 5'-terminal nucleotides is a DNA nucleotide. Even more preferably,
the probe has a DNA nucleotide in position 1 from the 5' end,
the probe has a DNA nucleotide in position 2 from the 5' end, or
the probe has DNA nucleotides in positions 1 and 2 from the 5' end.

Even more preferably, only the nucleotide at position 1 from the 5' end is a DNA nucleotide.

Accordingly, the 5 to 7 nucleotides at positions 1, 2, 3, 4, 5, 6, 7 and/or 8 from the 5' end may be LNA nucleotides. Preferably, at least the nucleotides at positions 4, 5 and 6 from the 5' end are LNA nucleotides, more preferably at least the nucleotides at positions 4, 5, 6 and 7 from the 5' end are LNA nucleotides, even more preferably at least the nucleotides at positions 4, 5, 6, 7 and 8 from the 5' end are LNA nucleotides, even more preferably at least the nucleotides at positions 3, 4, 5, 6, 7 and 8 from the 5' end are LNA nucleotides, even more preferably the nucleotides at positions 2, 3, 4, 5, 6, 7 and 8 from the 5' end are LNA nucleotides.

Most preferably, the nucleotide at position 1 from the 5' end is a DNA nucleotide and the nucleotides in positions 2, 3, 4, 5, 6, 7, and 8 from the 5' end are LNA nucleotides.

In each position, the probe may feature a determined nucleotide or a random nucleotide. The positions may be the same for all probes of a set of probes. Preferably, the composition comprises at least two set of probes. The composition may comprise one, two, three, four or more set of probes.

As used herein, the term "determined nucleotide" refers to a position of a certain nucleotide in the probe, wherein the type of the nucleotide (e.g., adenine (A), thymine (T), cytosine (C), guanine (G), uracil (U), 5-methylcytosine (mC)) is known. Preferably, the nucleobase at a determined position is A, T, C, G or U, more preferably, the nucleobase at a determined position is A, T, C or G.

Preferably, at the 5' end, the nucleotides are determined. More preferably,
at least nucleotide at position 1 from the 5' end is determined,
at least nucleotide at position 2 from the 5' end is determined,
at least nucleotide at position 3 from the 5' end is determined,
at least nucleotide at position 4 from the 5' end is determined,
at least nucleotide at position 5 from the 5' end is determined,
at least nucleotide at position 6 from the 5' end is determined,
at least nucleotides at position 1 and 2 from the 5' end are determined,
at least nucleotides at position 1 and 3 from the 5' end are determined,
at least nucleotides at position 1 and 4 from the 5' end are determined,
at least nucleotides at position 1 and 5 from the 5' end are determined,
at least nucleotides at position 1 and 6 from the 5' end are determined,
at least nucleotides at position 2 and 3 from the 5' end are determined,
at least nucleotides at position 2 and 4 from the 5' end are determined,
at least nucleotides at position 2 and 5 from the 5' end are determined,
at least nucleotides at position 2 and 6 from the 5' end are determined,
at least nucleotides at position 3 and 4 from the 5' end are determined,
at least nucleotides at position 3 and 5 from the 5' end are determined,
at least nucleotides at position 3 and 6 from the 5' end are determined,
at least nucleotides at position 4 and 5 from the 5' end are determined,
at least nucleotides at position 4 and 6 from the 5' end are determined,
at least nucleotides at position 5 and 6 from the 5' end are determined,
at least nucleotides at position 1, 2 and 3 from the 5' end are determined,
at least nucleotides at position 1, 2 and 4 from the 5' end are determined,
at least nucleotides at position 1, 2 and 5 from the 5' end are determined,
at least nucleotides at position 1, 2 and 6 from the 5' end are determined,
at least nucleotides at position 1, 3 and 4 from the 5' end are determined,
at least nucleotides at position 1, 3 and 5 from the 5' end are determined,
at least nucleotides at position 1, 3 and 6 from the 5' end are determined,
at least nucleotides at position 1, 4 and 5 from the 5' end are determined,
at least nucleotides at position 1, 4 and 6 from the 5' end are determined,
at least nucleotides at position 1, 5 and 6 from the 5' end are determined,
at least nucleotides at position 2, 3 and 4 from the 5' end are determined,
at least nucleotides at position 2, 3 and 5 from the 5' end are determined,
at least nucleotides at position 2, 3 and 6 from the 5' end are determined,
at least nucleotides at position 2, 4 and 5 from the 5' end are determined,
at least nucleotides at position 2, 4 and 6 from the 5' end are determined,
at least nucleotides at position 2, 5 and 6 from the 5' end are determined,
at least nucleotides at position 3, 4 and 5 from the 5' end are determined,
at least nucleotides at position 3, 4 and 6 from the 5' end are determined,
at least nucleotides at position 3, 5 and 6 from the 5' end are determined,
at least nucleotides at position 4, 5 and 6 from the 5' end are determined,
at least nucleotides at position 1, 2, 3 and 4 from the 5' end are determined,
at least nucleotides at position 1, 2, 3 and 5 from the 5' end are determined,
at least nucleotides at position 1, 2, 3 and 6 from the 5' end are determined,
at least nucleotides at position 1, 2, 4 and 5 from the 5' end are determined,
at least nucleotides at position 1, 2, 4 and 6 from the 5' end are determined,
at least nucleotides at position 1, 2, 5 and 6 from the 5' end are determined,
at least nucleotides at position 1, 3, 4 and 5 from the 5' end are determined,
at least nucleotides at position 1, 3, 4 and 6 from the 5' end are determined,
at least nucleotides at position 2, 3, 4 and 5 from the 5' end are determined,
at least nucleotides at position 2, 3, 4 and 6 from the 5' end are determined,
at least nucleotides at position 2, 3, 5 and 6 from the 5' end are determined,
at least nucleotides at position 1, 2, 3, 4 and 5 from the 5' end are determined,
at least nucleotides at position 1, 2, 3, 4 and 6 from the 5' end are determined,
at least nucleotides at position 1, 2, 3, 5 and 6 from the 5' end are determined,
at least nucleotides at position 1, 2, 4, 5 and 6 from the 5' end are determined,
at least nucleotides at position 1, 3, 4, 5 and 6 from the 5' end are determined,
at least nucleotides at position 2, 3, 4, 5 and 6 from the 5' end are determined, or
at least nucleotides at position 1, 2, 3, 4, 5 and 6 from the 5' end are determined.

Even more preferably at least the nucleotide at position 1 from the 5' end is determined, even more preferably at least the nucleotides at positions 1 and 2 from the 5' end are determined, even more preferably at least the nucleotides at positions 1, 2 and 3 from the 5' end are determined, even more preferably at least the nucleotides at positions 1, 2, 3 and 4 from the 5' end are determined, most preferably at least the nucleotides at positions 1, 2, 3, 4 and 5 or the nucleotides at positions 1, 2, 3, 4, 5 and 6 from the 5' end are determined.

Preferably, the random position(s) is/are located at position(s) 5; 6; 7; or 8 from the 5' end. More preferably, two random positions are located at positions 5 and 6, 5 and 7, 5 and 8, 6 and 7, 6 and 8 or 7 and 8 from the 5' end, three random positions are located at positions 5, 6 and 7 from the 5', positions 5, 6 and 8 from the 5', positions 5, 7 and 8 from the 5', positions 6, 7 and 8 from the 5', or four random positions at positions 5, 6, 7 and 8 from the 5'. Even more preferably, two random positions are located in positions 7 and 8 from the 5' end or three random positions are located in positions 6, 7 and 8 from the 5' end.

In a preferred embodiment, the composition of the present invention is characterized in that,
a) the set of probes has one LNA random position located at position 5; 6; 7; or 8 from the 5' end; or
b) the set of probes has two LNA random positions located at positions 5 and 6; 5 and 7; 5 and 8; 6 and 7; 6 and 8; or 7 and 8 from the 5' end, preferably at positions 7 and 8; or
c) the set of probes has three LNA random positions located at positions 5, 6 and 7; 5, 6 and 8; or 6, 7 and 8 from the 5' end, preferably at positions 6, 7 and 8.

The probes may preferably have the general structure of
5'-D-L-L-L-L-L-L-X-3',
5'-D-L-L-L-L-L-X-L-3'.
5'-D-L-L-L-L-X-L-L-3',
5'-D-L-L-L-X-L-L-L-3',
5'-D-L-L-L-L-L-X-X-3',
5'-D-L-L-L-L-X-L-X-3',
5'-D-L-L-L-X-L-L-X-3',
5'-D-L-L-L-L-X-X-L-3',
5'-D-L-L-L-X-L-X-L-3',
5'-D-L-L-L-X-X-L-L-3',
5'-D-L-L-L-L-X-X-X-3',
5'-D-L-L-L-X-L-X-X-3',
5'-D-L-L-L-X-X-L-X-3',
5'-D-L-L-L-X-X-X-L-3',
5'-L-D-L-L-L-L-L-X-3',
5'-L-D-L-L-L-L-X-L-3',
5'-L-D-L-L-L-X-L-L-3',
5'-L-D-L-L-X-L-L-L-3',
5'-L-D-L-L-L-L-X-X-3',
5'-L-D-L-L-L-X-L-X-3',
5'-L-D-L-L-X-L-L-X-3',
5'-L-D-L-L-L-X-X-L-3',
5'-L-D-L-L-X-L-X-L-3',
5'-L-D-L-L-X-X-L-L-3',
5'-L-D-L-L-L-X-X-X-3',
5'-L-D-L-L-X-L-X-X-3',
5'-L-D-L-L-X-X-L-X-3',
5'-L-D-L-L-X-X-X-L-3',
5'-L-L-D-L-L-L-L-X-3',
5'-L-L-D-L-L-L-X-L-3',
5'-L-L-D-L-L-X-L-L-3',
5'-L- L-D- L-X - L- L- L-3',
5'-L-L-D-L-L-L-X-X-3',
5'-L-L-D-L-L-X-L-X-3',
5'-L-L-D-L-X-L-L-X-3',
5'-L-L-D-L-L-X-X-L-3',
5'-L-L-D-L-X-L-X-L-3',
5'-L -L-D-L-X-X-L- L-3',
5'-L-L-D-L-L-X-X-X-3',
5'-L-L-D-L-X-L-X-X-3',
5'-L-L-D-L-X-X-L-X-3',
5'-L-L-D-L-X-X-X-L-3',
5'-L-L-L-D-L-L-L-X-3',
5'-L-L-L-D-L-L-X-L-3',
5'-L-L-L-D-L-X-L-L-3',
5'-L-L-L-D-X-L-L-L-3',
5'-L-L-L-D-L-L-X-X-3',
5'-L-L-L-D-L-X-L-X-3',
5'-L-L-L-D-X-L-L-X-3',
5'-L-L-L-D-L-X-X-L-3',
5'-L-L-L-D-X-L-X-L-3',
5'-L-L-L-D-X-X-L-L-3',
5'-L-L-L-D-L-X-X-X-3',
5'-L-L-L-D-X-L-X-X-3',
5'-L-L-L-D-X-X-L-X-3',
5'-L-L-L-D-X-X-X-L-3',
5'-L-L-L-L-D-L-L-X-3',
5'-L-L-L-L-D-L-X-L-3',
5'-L-L-L-L-D-X-L-L-3',
5'-L-L-L-L-X-D-L-L-3',
5'-L-L-L-L-D-L-X-X-3',
5'-L-L-L-L-D-X-L-X-3',
5'-L-L-L-L-X-D-L-X-3',
5'-L-L-L-L-D-X-X-L-3',
5'-L-L-L-L-X-D-X-L-3',
5'-L-L-L-L-X-X-D-L-3',
5'-L-L-L-L-D-X-X-X-3',
5'-L-L-L-L-X-D-X-X-3',
5'-L-L-L-L-X-X-D-X-3',
5'-L-L-L-L-X-X-X-D-3',
5'-L-L-L-L-L-D-L-X-3',
5'-L-L-L-L-L-D-X-L-3',
5'-L-L-L-L-L-X-D-L-3',
5'-L-L-L-L-X-L-D-L-3',
5'-L-L-L-L-L-D-X-X-3',
5'-L-L-L-L-L-X-D-X-3',
5'-L-L-L-L-X-L-D-X-3',
5'-L-L-L-L-L-X-X-D-3'.
5'-L-L-L-L-X-D-X-L-3',
5'-L-L-L-L-X-X-L-D-3',
5'-L-L-L-L-L-L-D-X-3',
5'-L-L-L-L-L-L-X-D-3',
5'-L-L-L-L-L-X-L-D-3',
5'-L-L-L-L-X-L-L-D-3',
5'-L-L-L-L-L-D-X-X-3', or
5'-L-L-L-L-X-L-X-D-3',
wherein D is a DNA nucleotide, each L is a LNA nucleotide and each X is a LNA random position.

More preferably, the probe may have the structure
5'-D-L-L-L-L-L-L-X-3',
5'-D-L-L-L-L-L-X-L-3',
5'-D -L-L-L-L-X-L- L-3',
5'-D-L-L-L-X-L-L-L-3',
5'-D-L-L-L-L-L-X-X-3',
5'-D-L-L-L-L-X-L-X-3'.
5'-D-L-L-L-X-L-L-X-3',
5'-D-L-L-L-L-X-X-L-3',
5'-D-L-L-L-X-L-X-L-3',
5'-D-L-L-L-X-X-L-L-3',
5'-D-L-L-L-L-X-X-X-3',
5'-D-L-L-L-X-L-X-X-3',
5'-D -L-L-L-X-X-L- X-3', or
5'-D-L-L-L-X-X-X-L-3',
wherein D is a DNA nucleotide, each L is a LNA nucleotide and each X is a LNA random position.

Even more preferably, the probe may have the structure
5'-D-L-L-L-L-L-L-X-3',
5'-D-L-L-L-L-L-X-X-3', or
5'-D-L-L-L-L-X-X-X-3',
wherein D is a DNA nucleotide, each L is a LNA nucleotide and each X is a LNA random position.

In the most preferred embodiment of the present invention, the probes have the general structure 5'-D - L-L-L-L-X - X - X-3', wherein D is a DNA nucleotide, each L is a LNA nucleotide and each X is a LNA random position.

In another highly preferred embodiment of the present invention, the probes have the general structure 5'-D-L-L-L-L-L-X- X-3', wherein D is a DNA nucleotide, each L is a LNA nucleotide and each X is a LNA random position.

The probes may be labeled. Preferably, the probes of different set of probes are labeled differently.

In a preferred embodiment of the present invention, the probes of first set of probes are labeled with a first marker and the probes of the second set of probes are labeled with a second marker, wherein the first marker is different from the second marker.

In the context of the present invention, the term "marker" may be understood interchangeably with "label" or "detectable moiety" as any molecule or moiety that enable the discrimination of the probe from other molecules by any means known in the art. As used herein, the first and/or the second marker may be selected from the group consisting of, but not limited to fluorescent markers, quenchers, non-fluorescent dyes, binding moieties, radioactive atoms (e.g., ³H ³²P, ³⁵S, ¹⁴C or lanthonoids), or heavy atoms (e.g., ²H or ¹³C).

In a more preferred embodiment, the first and the second marker are fluorescent markers.

As used herein, the terms "fluorescent marker", "fluorescence marker", "fluorescent label", "fluorescence label", "fluorescent dye", "fluorescence dye", "fluorophore" and "fluorescent moiety" may be understood interchangeably. A fluorescent marker may be understood in the broadest sense as a molecular moiety that emits light when it is excited by light of another wavelength. Typically the wavelength that is emitted by the fluorophore is shifted to longer wavelength in comparison to the excitation light. This shift is known as "Stokes-Shift" or "Stokes shift" to those skilled in the art. The Stokes shift can be less than 5 nm, more than 5 nm, more than 10 nm, more than 20 nm, more than 30 nm, more than 50 nm, more than 75 nm, more than 100 nm, more than 150 nm, more than 200 nm, more than 250 nm, more than 300 nm, or even more than 400 nm. The one or more absorbance maxima of the fluorophore suitable for fluorescent detection may at a wavelength from 100-280 nm (UV-C light), 280-315 nm (UV-B light), 315 nm-400 nm (UV-A light), 400-750 nm (visible light), 750-1400 nm (IR-A light), 1400-3000 nm (IR-B-light). The one or more excitation maxima of the fluorophore suitable for fluorescent detection may 100-280 nm (UV-C light), 280-315 nm (UV-B light), 315 nm-400 nm (UV-A light), 400-750 nm (visible light), 750-1400 nm (IR-A light), 1400-3000 nm (IR-B-light). Preferably, the absorbance and excitation maxima of the fluorescence are between 400 and 750 nm.

Fluorescent markers may be, e.g., fluorescein, fluorescein isothiocyanate (FITC), carboxyfluorescein, fluorescein derivatives, rhodamine dyes (Rhodamine, Rhodamine B, Rhodamine 6G, tetramethylrhodamine (TAMRA), rhodamine isothiocyanate and other Rhodamine derivatives) cyanine dyes (e.g., Cy3, Cy 3.5, Cy5, Cy5.5, Cy7) and derivatives thereof, LC dyes (e.g., LC-Yellow 555, LC-Red 610, LC-Red 640, LC-Red 670, LC-Red 705) and derivatives thereof, Alexa dyes (Alexa 488, Alexa 546, Alexa 647) and derivatives thereof, S0387, HOECHST dye and derivatives thereof, erythrosine isothiocyanate and derivatives thereof, Oregon Green and derivatives thereof, Lucifer Yellow and derivatives thereof, phycoerythrin and derivatives thereof, FAM and derivatives thereof, LightCycler® Yellow 555 and derivatives thereof, VIC and derivatives thereof, HEX and derivatives thereof or quantum dots and derivatives thereof.

In the context of fluorescent markers the term "derivative thereof" refers to salts of the fluorophore and(or fluorophores that are conjugated to non-fluorescent moieties such as, e.g. linkers (e.g., alkyl linkers, PEG linker, ) or binding moieties (e.g., maleimids, isothiocyanate or an active ester (e.g., succinimidyl ester, p-nitrophenylester, acid halogenides)) or a combination thereof.

The fluorescent markers may be linked to the probes by any means. They may be directly conjugated to the probes or conjugated via a linker. Such linker may preferably have the length of less than 5 A, less than 10 Å, less than 15 Å, less than 20 Å, less than 25 A or less than 50 Å. Various linkers are known to those skilled in the art (see, e.g., WO 84/03285). More preferably the linker length is between 15 and 35 Å.

Preferably, a prerequesite is that the linkage of the markers to the probe is not negatively influencing (which means that there is nearly or no significant (< 2°C) decrease in melting temperature) the melting temperature of a given probe with the target DNA. Therefore, the markers are attached to the nucleobase if the marker is linked to the DNA or LNA nucleotides or the marker is linked to an internucleosidic phosphate analog (WO 2007059816). Preferably, markers are attached to the 3' or 5' end of a probe. Such labeling methods are well known in the art and a plethora of commercial building blocks is available (see e.g. Fluorescent oligonucleotides. Versatile tools as probes and primers for DNA and RNA analysis. Wojczewski, Christian; Stolze, Karen; Engels, Joachim W. Synlett (1999), (10), 1667-1678).

The probe may be labeled with one, two, three, four, five or more fluorescent markers, preferably, the probe is labeled with one or two fluorescent markers.

When the probe is labeled with two fluorescent markers, these fluorescent markers may be the fluorescent markers of the same type or different fluorescent markers. Preferably, the fluorescent markers are different fluorescent markers. More preferably, the fluorescent markers emit and absorb light of a different wavelengths, even more preferably the two or more fluorescent dyes form a fluorescence resonance energy transfer (FRET) pair, even more preferably the emission wavelength of the donor fluorophore differs from that of the acceptor fluorophore in at least 25 nm, even more preferably at least 50 nm, at least 100 nm, at least 150 nm, at least 200 nm or at least 250 nm. Even more preferably the emission spectrum of one fluorophore (donor) overlaps with the absorbance spectrum of the other fluorophore (acceptor).

The probe may also be labeled with one or more fluorophore(s) and one or more quencher(s). Preferably, the probe may be labeled with one fluorophore and one quencher.

In an even more preferred embodiment of the present invention, the probes are hydrolysis probes additionally labeled with a quencher.

A quencher as used herein is a molecular structure that can quench the light emitted by a fluorophore. Therefore, a quencher that is in a comparably near special distance to a fluorophore can decrease the light intensity emitted by the fluorophore upon excitation. This effect is well-known by those skilled in the art. Also a fluorophore can, under certain circumstances, serve as a quencher.

The quencher may quench the light upon a spatial distance of less than 10, less than 9, less than 8, less than 7, less than 6, less than 5, less than 4, less than 3 or less than 2 nucleotides between the fluorophore and the quencher.

As used in the context of the present invention, the terms "quencher" and "dark quencher" may be understood interchangeably. A quencher may be any molecular structure that can efficiently decrease the intensity of the fluorescence emitted by the fluorophore. A quencher may be a fluorophore or a molecular structure not emitting visible light, such as e.g., Dabsyl (dimethylaminoazosulfonic acid), a Black Hole Quencher that quenches across the entire visible spectrum, an IRDye QC-1, a Qxl quencher, an Iowa black FQ that quenches in the green-yellow part of the spectrum, or an Iowa black RQ that quenches in the orange-red part of the spectrum. The quencher may emit thermal radiation.

In alternative sets of probes, the probes may be 7 (instead of 8) nucleotides in length. The probe and its application are as detailed above in the context of the compositions, methods and libraries of the invention, wherein the two or three LNA random positions of the probes being 8 nucleotides in length is reduced to one or two LNA random position(s), respectively, in the probes being 7 nucleotides in length by deleting one random position. The set of probes may be designed as described above considering the deletion of one random position. As mentioned above, these random nucleotides are preferably located at the 3' terminus.

Yet, in alternative sets of probes, the probes may be 9, 10, 11, 12, 13, 14, or 15 (instead of 8) nucleotides in length. The probe and its application are as detailed above in the context of the compositions, methods and libraries of the invention, wherein the number LNA random positions of the probes is increased in comparison to the probes being 8 nucleotides in length by introducing one or more additional random position(s), namely one additional random position for each nucleotide exceeding 8 (e.g. two additional random positions for a probe of 10 nucleotides). The set of probes may be designed as described above considering the addition of one or more random position(s). As mentioned above, these random nucleotides are preferably located at the 3' terminus.

Alternatively, the term "nucleotide" may also comprise another nucleobase than A, T, C or G as known in the art (such as, e.g., uracil (U) or methyl cytosine (mC)) conjugated to a molecular moiety that can polymerize. Therefore, the term "nucleotide" may alternatively refer to a ribonucleic acid (RNA) nucleotide, a nucleic acid analogue nucleotide, as known to those skilled in the art (e.g., a peptide nucleic acid (PNA) nucleotide, a Morpholino nucleotide, glycol nucleic acid (GNA) nucleotide, a threose nucleic acid (TNA) nucleotide or a methylated DNA nucleotide. A nucleic acid analogue nucleotide is selected in such a manner that it is capable of forming a stable and specific base pair with a natural base. Alternatively, the locked nucleic acid (LNA) may also be a part of a strand comprising at least one LNA nucleotide and at least one DNA nucleotide and further comprising one of the following: a ribonucleic acid (RNA) nucleotide, a peptide nucleic acid (PNA), a Morpholino, a glycol nucleic acid (GNA), threose nucleic acid (TNA), ENA (2'-O,4'-C-ethylene-bridges nucleic acid) and 2'-amino-LNA derivatives. Alternatively, said at least one DNA nucleotide may also be replaced by one of the aforementioned nucleotides. Alternatively, said at least one LNA nucleotide may also be replaced by one of the aforementioned nucleotides.

In a second aspect, the present invention relates to a method of determining the genotype at a locus of interest in a sample obtained from a subject, the method comprising
a) contacting the sample comprising genetic material with the composition of the present invention; and
b) detecting the binding of a probe of the first or the second set of probes to the genetic material, thereby determining the genotype at the locus.

As used herein, the term "determining the genotype" refers to the analysis of the genotype of an organism or a virus. Determining the genotype may include, but may not be limited to the analysis of an allele (e.g., the analysis whether the organism has a point mutation in a certain locus or not, and which nucleotide or nucleobase has been replaced by which other nucleotide or nucleobase), the search for novel point mutation(s) in the genome of an organism or virus, or the determination or assessment of the copy number of a gene or a part of a gene.

In the context of the present invention, the term "locus of interest" may be understood interchangeably with the term "locus" in the broadest sense as a position in the nucleotide sequence of a gene, in particular the target gene to which the probe of the invention may bind. The locus of interest may comprise one, two, three, four, five, six, seven or eight nucleotide(s). There may be a mutation localized at the locus of interest, or there may be no mutation localized at the locus of interest. There may be a single nucleotide polymorphism (SNP) localized at the locus of interest, or there may be no single nucleotide polymorphism (SNP) at the locus of interest.

The term "subject" as used herein may be understood in the broadest sense as a source of genetic material. The genetic material may be obtained from any organic material, in particular a biological sample. The biological sample may be a living or dead organism, such as, e.g., a living or dead bacterium, a living or dead animal, a living or dead human, a living or dead fungus or a living or dead plant, a part of an animal, a plant, a fungus, or a cell organelle a virus, a virus-like particle such as a mitochondrium, a leucoplast or a chloroplast. Alternatively, DNA or RNA may be obtained from an expulsion, a scale off or a degradation product of the aforementioned organisms. It may be obtained, e.g., from a blood sample, an embryo blood sample, a fetal blood sample, a lymph sample, a cord blood sample, a *liquor cerebrospinalis* sample, an amniotic liquor/fluid sample, a mouth swab, a vaginal swab, a smear test, a dander, a hair follicle, one or more extracted cells, a sperm sample, an ovule cell, a saliva sample, a urine sample, a stool sample, a lymph sample, a sanies sample, a umbilical cord sample, a skin sample, a bone marrow sample, a mucosa sample, a tissue sample, a sample of water, a sample of soil, a sample of sediment, or a crime scene.

The term "genetic material" may refer to any kind of natural or synthetical nucleic acid that conveys or encodes genetic information by a sequence of nucleobases.. The genetic material may be DNA or RNA. DNA may be double-stranded DNA or single stranded DNA. RNA may be any kind of RNA known in the art such as, e.g., mRNA, tRNA, ribosomal RNA, viral RNA, miRNA siRNA, RNAi, snRNA. The genetic material may be obtained from a biological sample or may be synthesized by organic synthesis. As used herein, the term "sample" refers to any kind of material that is analyzed by means of the probe of the present invention. The sample may comprise the genetic material of interest.

The sample may be a biological sample selected from the group consisting of a body fluid, blood, urine, serum, mucosa, sputum feces, epidermal sample, skin, cheek swab, sperm, amniotic fluid, cultured cells and bone marrow. Further, the genetic material may alternatively be obtained by chemical synthesis as known in the art. It may be part of a naturally occurring genome or a genome of a genetically modified organism. It may be linear or circular genetic material, in particular genomic DNA or a plasmid. The DNA or RNA material may also be purified.

The sample may be obtained from an animal. The animal may be any kind of animal, including unicellular animals (*protozoa*) and multicellular animals (*metazoa*). Preferably, the animal is a mammal, including humans. The term "animal" may also include spores or cysts of a protozoan and cysts and gametes (germ cells) of a metazoan.

Alternatively, the sample may be obtained from a plant. The plant may be any kind of plant, including unicellular and multicellular plants. Preferably, the plant is a useful plant, such as an agricultural crop. The term "animal" may also include spores of a protozoan and seeds, fruit, fallen leaves, pollen, sap, gametes (germ cells) of a metazoan plant. Alternatively, the sample may be obtained from a bacterium. The bacterium may be any kind of protozoa, including *eubacteria* and *archaebacteria.* The bacterium may or may not be pathogenic. Preferably, the bacterium is a pathogenic bacterium. The term "bacterium" may also include spores of a bacterium.

Alternatively, the sample may be obtained from any kind of virus. The virus may also include animal and plant viruses and phages. The virus may be a virus particle (virion). Alternatively, the virus DNA and/or RNA may be obtained from virus genome that is present in a eukaryotic or a prokaryotic cell (host cell). The virus genome may be integrated in the host cell's genome. Preferably, the virus is a pathogenic virus.

Alternatively, the sample may be obtained from a fungus. The fungus may be any kind of fungus. Preferably, the fungus is a pathogenic virus, parasiting in or on the surface of a host organism, in particular in or on the surface of an animal, including humans, or a plant. The term "fungus" may also include spores of a fungus.

The DNA or RNA obtained from a biological sample may be comprised in a crude biological mixture or may be isolated. The term "crude biological mixture" may include, but may not be limited to a cell lysate that may be obtained by any means known in the art (such as lysis by means of, e.g., a hypotonic buffer, detergent(s), sonication, alcohol, shear forces (by means of a French Press, a Potter or Downs homogenisator, rough pipetting), enzymes, scratching or freeze-thaw-cycle(s) ("freeze-and-squeeze")) and DNA- or RNA-containing smear. The term "isolated" may refer to dried or freeze-dried sample that, apart from salts, contains more than 25 %, more than 50 %, more than 60 %, more than 70 %, more than 80 %, more than 90 %, more than 95 % or more than 97 % by weight DNA and/or RNA, or an aqueous or organic solution, wherein at least 25 %, at least 50 %, at least 60 %, at least 70 %, at least 80 %, at least 90 %, at least 95 % or at least 97 % by weight of the organic content, apart from the solvent and salts, refers to DNA and/or RNA.

The DNA or RNA may be isolated by any means known in the art, such as, e.g., precipitation in alcohol (e.g., ethanol, isopropanol) or a mixture of different alcohol(s), chromatography-based methods (e.g, anion exchange chromatography, size exclusion chromatography), gel-based methods (e.g., gel electrophoresis (agarose gel electrophoresis, polyacrylamide gel electrophoresis (PAGE))), capillary electrophoresis (CE) or combinations of two or more thereof. Several purification methods may be combined on-line or may be combined by two or more subsequent purification steps. Herein, the terms "isolation" and "purification" may be understood interchangeably.

The DNA or RNA may also be of synthetical origin. In particular, a control sample may be of synthetical origin. The control sample may be a positive or a negative control, or may be used to test the selectivity of the probes. Synthetical DNA may be linear or circular. Target DNA or target RNA of synthetical origin may be labeled by any means, in particular by means described for the probes herein. The synthetical DNA may be double-stranded or single-stranded. It may also be conjugated to a surface, in particular to the surface of a bead or the surface of a plastic, glass or metal slide. The DNA or RNA may be part of an array, in particular a microarray, in more particular a DNA microarray (e.g., an SNP array or an Affymetrix chip, array comparative genomic hybridization) as known in the art.

The target sequence may be part of a target DNA or of a target RNA. The target sequence may be freely chosen. For instance, the sequence of the determined nucleotides may be known by the person skilled in the art or be obtained from a sequence database as known by those skilled in the art, such as, e.g., the Ensembl Sequence Identifier, the RefSeq, the EMBL Sequence Identifier, the EMBL and/or the NCBI Entrez Genome database. Additionally, the probe design may be computer-assisted, such as, e.g., by the web-based ProbeFinder Assay Design Software or LightCycler Primer Design Software (both from Roche).

The term "target DNA" as used herein may be understood as the DNA that is at least in part complementary to the probe of the invention. Therefore, under certain conditions, the probe can bind to its target DNA. It may be understood that the probe preferably binds to the complementary single strand DNA. A single strand DNA may be obtained by means known in the art, such as melting a double-stranded DNA strand as described further herein.

The target DNA may be of any length. Preferably, the target DNA is at least 8 nucleotides in length. The target DNA may be more than 8, more than 9, more than 10, more than 20, more than 50, more than 10², more than 10³, more than 10⁴, more than 10⁵, more than 10⁶, more than 10⁷, more than 10⁸, more than 10⁹, more than 10¹⁰ or more nucleotides in length. It may be understood by a person skilled in the art that, in the context of double-stranded DNA, the term "nucleotides in length" may also refer to the length in base pairs (bp).

The target RNA may be of any length. Preferably, the target RNA is at least 8 nucleotides in length. The target RNA may be more than 8, more than 9, more than 10, more than 15, more than 20, more than 30, more than 50, more than 75, more than 100, more than 150, more than 200, more than 300, more than 500, more than 1000, more than 2000 or more nucleotides in length. It may be understood by a person skilled in the art that, in the context of double-stranded RNA, the term "nucleotides in length" may also refer to the length in base pairs (bp).

The target DNA may be amplified by any means in the art. Such as, e.g., polymerase chain reaction (PCR), amplification in bacteria, in yeast, in mammalian cells or in insect cells. Further, DNA may be obtained from a reverse transcriptase reaction, such as e.g. reverse transcriptase polymerase chain reaction. Reverse transciptase-PCR may also be used to analyze the transcriptome or a particular messenger RNA (mRNA).

The set of probes may be designed to characterize the genotype of the target gene. One set of probes may have a stronger binding affinity to a locus of a wildtype genotype than the mutated genotype, whereas a corresponding set of probes may have a stronger binding affinity to the locus of the mutated genotype. Therefore both set of probes bind preferably to different alleles of the same locus.

Herein, the term "corresponding set of probes" refers to a probe that has an identical nucleotide sequence with the exception of the base(s) at one two or three LNA random position(s) and the base at the discriminating position than the probe it corresponds to.

As used in the context of the present invention, the term "contacting" may be understood in the broadest sense as the exposure of one or more sets of probes to the sample and *vice versa.* The contacting may be achieved, e.g., by ad mixing a solution comprising the one or more sets of probes to a sample. Optionally, contacting may be accompanied by a heating step:
As used throughout the invention, the term "detecting" may be understood in the broadest sense as the measurement of a signal occurring from a probe. The probe may be labeled with a marker and a signal occurring from the marker may be detected. Therefore, e.g., the fluorescence signal occurring from an excited fluorescently labeled probe may be detected. Alternatively, the fluorescence resonance energy transfer (FRET) signal occurring from a doubly labeled probe, the radioactive radiation occurring from a radioactively labeled probe, the melting temperature of the probe, the fluorescence depolarization of a fluorescently labeled probe, and/or the diffusion speed of a fluorescently labeled probe may be detected. Alternatively, a FRET signal and/or a fluorescence cross-correlation (FCCS) signal of two fluorescent labeled probes may be detected. These probes may interact with each other or with one target nucleotide molecule or may interact with two complementary DNA strands. Alternatively, the fluorescence quenching may be detected by the loss, decrease or absence of fluorescence.

In the context of the present invention, the binding of the first set of probes may be compared to the binding of the second set of probes. Herein, after contacting the sets of probes with a sample, the intensity of the signal occurring from the first set of probes may be detected. Likewise, the signal occurring from the second set of probes may be detected. The signal intensities may be compared with another.

The first and the second set of probes can be labeled differently. Then, the signal occurring from both sets of probes may be detected contemporaneously. The signal occurring from each one marker may be detected separately. For example, both sets of probes may be labeled with two different fluorophores emitting light of different wavelengths. The emitted light may be detected independently from another by any means known in the art, e.g., by separate fluorescence detectors (e.g., photomultiplier tubes (PMTs), avalanche photodiodes (APDs)), or may be detected by a single detector, but the light of the different wavelength is separated from another, e.g. by one or more filter(s), one or more dichroic mirror(s), one or more prism(s) or a Meta detector.

. Alternatively both sets of probes may be labeled by the same marker, e.g., the same fluorophore. Then, the signal occurring from both sets of probes may be detected subsequently.

As mentioned herein, the first and the second set of probes may differ in the discriminating position. Preferably, the first and the second set of probes may differ in a single nucleotide only. However, due to the difference in their nucleotide sequence, the two set of probes may have different binding affinity when binding to their target sequence. The probe having a full match to the complementary nucleotide strand may have a higher affinity than the probe bearing a mismatch. Preferably one of the probes of one set of probes shows a fulmatch with the target strand, thus, all nucleobases of the form base pairs with the nucleobases of the target strand, whereas the probes of the second set of probes bear at least a single mismatch.

A mismatch will lead to a comparably lower binding affinity than a fulmatch. Thus, in an equilibrium, a larger fraction of the set of probes bearing the sequence that fits better will bind. Therefore, binding of the probe showing fulmatch can be identified by obtaining an altered detection signal from the probe showing binding.

However, the person skilled in the art will notice that the target nucleotide may also have a different sequence at a certain locus.

Therefore, as described herein, one of the sets of probes may be designed to have a preference for binding to a specific genotype at the locus, whereas the other set of probes may have a preference for another genotype at the locus. Therefore, when comparing the detection signal of both probes, the genotype at the locus may be determined.

As used herein, the term "determining the genotype at the locus" may refer to the discriminating of different genotypes in a locus of interest in two or more different samples. Alternatively, the term "determining the genotype at the locus" may refer to the identification of the genotype of one sample. Further, the term "determining the genotype at the locus" may also refer to the identification of novel genotype variants in the genome of a subject.

For instance, the genotype of a subject, in particular a patient, may be determined by the method of the present invention to select a certain therapy. The determination of genotypes by the method of the present invention may also be used for epidemiologic screenings of a population. Further, the determination of a genotype by the method of the present invention may also be used for disease provision of a subject, for detecting a certain plant species or strain, for detecting a certain bacterial species or strain, for detecting a certain viral species or strain, for detecting a certain fungal species or strain, for detecting a certain animal species, strain, race or breed, or for criminal investigations. Further, the determination of a genotype by the method of the present invention may also be used for the detection of genotypic characteristics of animals, plant, viruses, bacteria and/or fungi. As known in the art, genotypic characteristics may have influences on the phenotype. Therefore, the method may be used to predict phenotypic characteristics. Further, the method may be used to detect a predisposition of a disease in an animal, including human, or a plant, or a fungus. Further, the method of the present invention may be used for prenatal diagnostics. Herein, the sample may preferably be obtained from the blood or the lymph of the embryo or fetus, the cord blood, the placenta or the amniotic liquor/fluid.

In a preferred embodiment of the present invention, the locus is a single nucleotide.

A single nucleotide in a particular position of the target DNA or target RNA may be of interest. Therefore, there may be a single nucleotide polymorphism (SNP) in the locus.

In a further preferred embodiment, the method comprises
(a) performing an amplifying step comprising contacting the sample with a set of primers to produce an amplification product including the locus of interest,
(b) performing a hybridizing step comprising contacting the amplification product of step a) with the composition of the present invention; and
(c) detecting the hybridizing of a probe of the first or the second set of probes to the genetic material thereby determining the genotype at the locus.

The term "amplifying step" as used herein refers to any method known in the art for amplifying genetic material. DNA may be amplified by using polymerase chain reaction, may be amplified in cells (e.g., in bacteria cells, in mammalian cells, in insect cells). RNA may be amplified by using reverse transcriptase PCR.

The term "contacting the sample with a set of primers" refers to the addition of primers to the sample comprising the target DNA. Further enzyme(s) such as DNA polymerase, magnesium salt(s), nucleotides triphosphates (dATP, dCTP, dTTP, dGTP) and/or a suitable buffer may be added. These ingredients are well known by those skilled in the art and commercially available.

The term "hybridizing step" may refer to any method known in the art for hybridizing the probe with its target DNA or target RNA. The probes may hybridize with their target DNA or target RNA under conditions a short nucleic acid regularly hybridizes with its target sequence. Such conditions are well-known by those skilled in the art.

For hybridization, double-stranded DNA may be first denaturated, thus, both DNA strands may be separated from another. The target DNA may be denaturated, e.g., by heating the sample. The target DNA strand may be denaturated at more than 40°C, more than 50°C more than 60°C, more than 70°C, more than 80°C, more than 90°C, more than 95°C, more than 96°C, more than 97°C, more than 98°C or more than 99°C. Preferably, the target DNA strand may be denaturated at more than 60°C, more than 70°C, more than 80°C, more than 90°C, more than 95°C, more than 96°C, more than 97°C, more than 98°C or more than 99°C. By denaturating the DNA strand, the two strands on the double helix are separated. This process may be also designated as "melting" of DNA.

In a solution comprising the target DNA and the respective probe as used in the present invention, the probe may anneal with the DNA strand when cooling said solution down to less than 75°C, less than 70°C, less than 65°C, less than 60°C, less than 55°C, less than 50°C, less than 45°C, less than 40°C, less than 39°C, less than 38°C or less than 37°C.

The term "amplification product" may be understood as the product of the amplification step as described herein. Typically, the amplification product is shorter than the target DNA, but longer than the probe. The locus of interest may be included in the amplification product.

The probe may also be used for a polymerase chain reaction (PCR) as known in the art. Herein, the probes may be labeled or unlabelled. Preferably, both probes are labeled. The two probes may be labeled with two different fluorophores. Alternatively, one probe may be labeled with a fluorophore and the other probe may be labeled with a quencher. One probe may also be labeled with two different fluorophores, or with one fluorophore and one quencher. The probe(s) and/or primer(s) are comprised in a solution further comprising the target DNA, a buffer (comprising water and optionally comprising a pH buffer, a magnesium salt and cofactors of the DNA polymerase), DNA polymerase, a nucleotide mixture (comprising dATP, dGTP, dCTP and dTTP).

First, the target DNA strand is denaturated. For instance, depending on its length, a target DNA strand may be denaturated as described above. By denaturating the DNA strand, the two strands on the double helix separate.

Then, the solution is cooled down to less than 75°C, less than 70°C, less than 65°C, less than 60°C, less than 55°C, less than 50°C, less than 45°C, less than 40°C, less than 39°C, less than 38°C or less than 37°C.

At the temperature optimum of the used DNA polymerase that may preferably in the range of 50 to 80°C, more preferably in the range of 50 to 65°C, the DNA strand is elongated. Then the next cycle may start by denaturation of the DNA strands. Detection may be in real-time.

The PCR reaction may be conducted by any means known in the art. It may be conducted manually or automatized. Automatized PCR may be conducted on a standard PCR machine or on a real-time PCR machine (e.g. a LightCycler®). The PCR may be quantitative PCR (qPCR). The PCR may be reverse transcriptase PCR (RT-PCR). Different PCR methods may also be combined with another. The PCR reaction may be combined by detection of a FRET effect. Detection may be in real-time.

In a further preferred embodiment, the method is characterized in that,
(i) the detecting is by measuring presence or absence of fluorescence;
(ii) the detecting is in real-time;
(iii) the marker is selected from the group consisting of fluorescein, LC-Yellow 555, FAM, VIC, HEX, Rhodamine B, Rhodamine 6G, LC-Red 610, LC-Red 640, LC-Red 670, LC-Red 705, Cy3, Cy 3.5, Cy5, Cy5.5 and a quencher;
(iv) the amplifying step employs a polymerase enzyme having 5' to 3' exonuclease activity; and/or
(v) the sample is a biological sample, preferably a sample selected from the group consisting of a body fluid, a blood sample, a urine sample, serum, mucosa, sputum feces, epidermal sample, skin sample, cheek swab, sperm, amniotic fluid, cultured cells and bone marrow sample.

The probe may be labeled with a marker, in particular a fluorescent marker such as, e.g., fluorescein dyes (e.g., fluorescein, fluorescein isothiocyanate (FITC), rhodamine dyes (Rhodamine, Rhodamine B, Rhodamine 6G, tetramethylrhodamine (TAMRA), rhodamine isothiocyanate) cyanine dyes (e.g., Cy3, Cy 3.5, Cy5, Cy5.5, Cy7), LC dyes (e.g., LC-Yellow 555, LC-Red 610, LC-Red 640, LC-Red 670, LC-Red 705), Alexa dyes (Alexa 488, Alexa 546, Alexa 647), S0387, HOECHST dye, erythrosine isothiocyanate, Oregon Green, Lucifer Yellow, VS, phycoerythrin, FAM, LightCycler® Yellow 555, VIC, HEX or quantum dots.

The fluorophore may be excited with light of a wavelength near to one of its absorbance maxima. Alternatively, the fluorophore may be excited by light of a high intensity of the double wavelength of one of its absorbance maxima (two photon effect).

The probe may be labeled with one, two, three, four, five or more fluorescent markers, preferably, the probe is labeled with one or two fluorescent markers.

When the probes are labeled with two fluorescent markers, these fluorescent markers may be the fluorescent markers of the same type or different fluorescent markers. Preferably, the probe is labeled with two fluorescent markers, wherein the emission spectrum of donor fluorophore overlaps with the absorbance spectrum of the acceptor fluorophore. Even more preferably, the probe is labeled with two fluorophores that enable FRET (fluorescence resonance energy transfer).

The donor fluorophore may absorb light at a shorter wavelength than the acceptor fluorophore. Further, the donor fluorophore preferably emits light at a shorter wavelength than the acceptor fluorophore. Even more preferably, the emission spectrum of the donor fluorophore largely overlaps with the absorbance spectrum of the acceptor fluorophore. Alternatively, the maximum of the excitation spectrum of the donor fluorophore may be at the double wavelength of the absorbance maximum of the acceptor fluorophore. Then, a two-photon system is used for FRET.

The FRET technology is well-known to those skilled in the art (see, e.g., U.S. patent Nos. 4,996,143, 5,565,322, 5,849,489, and 6,162,603). It is based on a concept that energy is transferred from a donor fluorophore to an acceptor fluorophore that on its part emits light. Upon irradiation of the donor fluorophore with a certain wavelength that is absorbed by the donor fluorophore the donor fluorophore excited. If no acceptor fluorophore is in its near special distance, the donor fluorophore emits light of a certain red-shifted wavelength (bathochrome effect). But, if an acceptor fluorophore is in its near special distance, FRET occurs. The energy transfers from the donor fluorophore to the acceptor fluorophore occurs by resonance energy transfer (Förster energy transfer), thus, preferably without emitting light. Hereby, the acceptor fluorophore is excited and may emit light. The light emitted by the acceptor fluorophore is further red-shifted (bathochrome shift, Stokes shift). The stokes shift of the used fluorophores may preferably be more than 20 nm, more than 30 nm, more than 40 nm, more than 50 nm, more than 75 nm, more than 100 nm, more than 125 nm, more than 150 nm or more than 200 nm.

The occurrence of a FRET effect can be determined and quantified in different ways. The decrease of the light intensity emitted by the donor fluorophore upon the occurrence of FRET may be quantified. Alternatively or additionally, the increase of the light intensity emitted by the acceptor fluorophore upon the occurrence of FRET may be quantified.

The FRET intensity depends on the special distance between the donor and the acceptor fluorophore. The spatial distance on the probe may preferably be less than 10, less than 9, less than 8, less than 8, less than 7, less than 6, less than 5, less than 4, less than 3, or less than 2 nucleotides. Preferably, the spatial distance between the donor and the acceptor fluorophore is within the Förster radius.

The donor fluorophore may be excited with any kind of light source. Preferably, the light source emits light of a defined wavelength. The light source may be, e.g., an argon ion laser, a high intensity mercury (Hg) arc lamp, an LED diode, a HeNe laser or a HeCd laser. The excitation light may be directed through one or more filter(s) and/or one or more dichroic mirror(s) selecting the desired wavelength. Likewise, the emitting light may be directed through one or more filter(s) and/or one or more dichroic mirror(s) selecting the desired wavelength.

The FRET experiment may be carried out on any experimental setup known in the art for quantifying FRET intensity. The experimental setup may comprise, e.g., a photon counting epifluorescent microscope (containing the appropriate dichroic mirror(s) and filter(s) for monitoring fluorescent emission at the particular range), a photon counting photomultiplier system or photometer.

Detecting the FRET may conducted by measuring presence or absence of fluorescence. As used in the context of the present invention, the term "absence of fluorescence" refers to a fluorescence rate of than 20 %, less than 15 %, less than 10 %, less than 5 %, less than 4 %, less than 3 %, less than 2 %, less than 1 % or less than 0.5 % of the fluorescence intensity that is maximally found for the same fluorophore under the same conditions (such as in the same buffer, at same temperature, with the same excitation intensity on the same apparatus with the same settings).

For an intact probe, a strong FRET effect may be detectable. For a cleaved probe or a probe of which one or two fluorophores were cleaved off, no or a far lower FRET effect may be detectable. Therefore a FRET probe may be a hydrolysis probe, such as, e.g., a TaqMan® probe, as described herein.

Alternatively, the occurrence of two fluorophores on one molecular structure may also be determined by fluorescence cross-correlation spectroscopy (FCCS). Herein, it may be determined whether the fluorescence signals occurring from two different fluorophores of a doubly labeled probe diffuse conjointly or independently. Alternatively, it may be determined whether the fluorescence occurring from two different fluorophores of two differently labeled probes binding to the same target DNA diffuse conjointly or independently.

Further, an amplified luminescence proximity homogenous assay (ALPHA, AlphaScreen) may be used.

The probes can also be labeled with one fluorophore only. Then, the binding to the target sequence may be detected by measuring the fluorescence depolarization or the diffusion speed. The term "diffusion speed" may include the lateral and tangential diffusion velocity, the rotational speed of the molecule, the intramolecular rotational speed, any kind of molecular oscillation and combinations thereof.

Fluorescence depolarization bases on fluorescence anisotropy. In fluorescence depolarization assays the rotational diffusion of a molecule is determined from the decorrelation of polarization in fluorescence, i.e., between the exciting and emitted (fluorescent) photons. This decorrelation may be measured as the "tumbling time" of the molecule as a whole, or of a part of the molecule relative to the whole. From the rotational diffusion constants, the experimenter may determine whether fast tumbling low-molecular weight probe has bound to its slowly tumbling high-molecular weight target sequence.

The diffusion speed may be measured by fluorescence correlation spectroscopy (FCS) as known to those skilled in the art. Herein, the dwelling time of freely diffusion molecules is measured. From the average dwelling time (diffusion constant), the experimenter may determine whether fast diffusing low-molecular weight probe has bound to its slowly diffusing high-molecular weight target sequence or the low-molecular weight probe is diffusing freely.

The probe may also be labeled with one or more fluorophore(s) and one or more quencher(s). Preferably, the probe may be labeled with one fluorophore and one quencher.

In an even more preferred embodiment of the present invention, the probes are hydrolysis probes additionally labeled with a quencher.

A quencher as user herein is a molecular structure that can quench the light emitted by a fluorophore. Therefore, a quencher that is in a comparably near special distance to a fluorophore can decrease the light intensity emitted by the fluorophore upon excitation.

The quencher may quench the light upon a spatial distance of less than 10, less than 9, less than 8, less than 7, less than 6, less than 5, less than 4, less than 3 or less than 2 nucleotides between the fluorophore and the quencher.

The probe can also be a TaqMan® probe, a molecular beacon, a scorpion primer or a lux primer as known in the art. Preferably, the probe is a TaqMan® probe.

Alternatively, the probes may be labeled with a non-fluorescent dye such as, e.g., a p-nitrophenyl moiety or Malachite Green, or with reactive small molecules that can bind to other molecules, such as, e.g., maleimides, isothiocyanates or active esters (e.g., succinimidyl esters, p-nitrophenyl esters). Further, the probes may be labeled with small molecules selectively binding to high-molecular weight molecules, such as, e.g., biotin methotrexate or glycocorticoids. A probe labeled with biotin may be detected by using labeled strepavidine. A probe labeled with methotrexate may be detected by using labeled dihydrofolate reductase (DHFR). A probe labeled with a glycocorticoid may be detected by using labeled a labeled antibody or antibody derivatives (e.g., Fab fragments, single chain antibodies, diabodies, triabodies, and tandabs) directed against said glycocorticoids. Alternatively, the probe may be unlabeled and may be detected by a labeled antibody or a labeled antibody derivative (e.g., a Fab fragment, a single chain antibody, a diabody, a triabody, a tandab). Alternatively, the probe may be unlabeled and may be detected by an unlabeled antibody or an unlabeled antibody derivative (e.g., a Fab fragment, a single chain antibody, a diabody, a triabody, a tandab) that is on its part detected by a labeled antibody or antibody derivative directed against its Fc part.

Further, the probe may be conjugated with an enzyme that can generate a color from a precursor (e.g., a peroxidase, alkaline phosphatase). This conjugation may be obtained by conjugating the probe covalently to the enzyme or by conjugating the probe with a binding molecule (e.g., digoxigenine, methotrexate) that can bind to a fusion protein of the enzyme enzyme that can generate a color from a precursor and a protein binding to the binding molecule. Alternatively, the enzyme may be able to emit light by chemical conversion (chemoluminescence) (e.g., luciferase).

Alternatively, the probes may be radioactively labeled. Therefore, the probe may be labeled with, e.g., ³H, ³²P, ³⁵S, ¹⁴C lanthonoids or other radioactive labels.

Alternatively, the probes may be labeled with heavy atoms detectable by nuclear magnetic resonance (NMR) or mass-spectrometry (e.g., ESI- or MALDI-MS). Therefore, the probe may be labeled with, e.g., ²H or ¹³C.

Moreover, the melting point of the probe(s) may be determined. It may be understood by those skilled in the art that a higher melting point refers to a stronger binding. Thus, a probe binding stronger to its target sequence than a corresponding probe bearing one or more altered nucleotide positions has lesser mismatches with the target sequence than the corresponding probe. Thereby the genotype of one or more samples may be characterized.

Preferably, one of the probes of one of the two or more sets of probes shows a fulmatch with the target sequence, the other probes of the same set of probes show one or more mismatch(es) with the target sequence. The probes of the corresponding set(s) of probes may show an additional mismatch in the discriminating position.

The probes may be TaqMan® probes. TaqMan® probes may be used to conduct a TaqMan® assay. The TaqMan® assay is well known to those skilled in the art. As used herein, the terms "TaqMan® probe" and "hydrolysis probe" may be understood interchangeably. The TaqMan® probe comprises a fluorophore and a quencher. Preferably, the fluorophore and the quencher are located near the termini of the probe, more preferably, the fluorophore is located near the 5' terminus, and the quencher is located near the 3' terminus. The term "3'-terminal" may be understood in the broadest sense as understood in the art. Further, the terms "3' terminus" and "3' end" may be understood interchangeably as known in the art. The person skilled in the art will notice that the terms "3' terminus" and "3' end" as used herein may refer to the 5' end of the nucleotide strand, but may not exclude that at the 3' end another molecular moiety (such as, e.g., a fluorophore, a quencher, a binding moiety or the like) is added to the 3' end of the probe.

As used herein, the term "located near" preferably means that the fluorescence or quencher moiety is located not more than 4, more preferably not more than 3, even more preferably not more than 2, and most preferably at the first nucleotide from the respective terminus.

The TaqMan® probe may hybridize to its target sequence. Further, the used composition may further comprise a pair of primers, thus one forward and one reverse primer. These primers are preferably unlabeled. Preferably, the forward primer binds upstream, the reverse primer downstream of the band, such that the TaqMan® probe binds to a sequence that is a part of the strand that is amplified. A PCR reaction as well-known in the art is conducted. Thus, the target DNA is melted, then conditions are chosen that enable the annealing of the primers and the probe to the target DNA. Subsequently, conditions are chosen that enable the DNA polymerase to amplify the DNA strand between the primers. In the context of the TaqMan® assay, the DNA polymerase preferably has a 5' to 3' exonuclease activity. More preferably, the DNA polymerase is Taq polymerase or a functional variant thereof. When the DNA polymerase comes to the TaqMan® probe, the 5' end is cleaved off. Thereby, the fluorophore or quencher bound to the 5' terminal nucleotide(s) is also cleaved off. Preferably, the fluorophore is cleaved off. Consequently the fluorophore and the quencher may diffuse in different directions. The spatial distance between both may be significantly increased and the fluorescence occurred by the fluorophore is significantly increased as it is not quenched by the dark quencher any longer. Preferably, the TaqMan® assay is analyzed in real-time. More preferably, the TaqMan® assay is conducted during a life-time PCR method. It may also be conducted quantitatively in a qPCR reaction.

The TaqMan® assay using the probes of the present invention may preferably be used for the discrimination of alleles, genotyping, bacterial identification assays, DNA quantification, and the determination of the viral load in clinical specimen, gene expression assays and verification of microarray results. More preferably it may be used for the discrimination of alleles, genotyping and bacterial identification assays. Genotyping may preferably be single nucleotide polymorphisms (SNP) genotyping, therefore, the determination of a genotype at defined a locus of interest in a sample, wherein the locus is a single nucleotide. Alternatively, genotyping may be copy number variant (CNV) genotyping. A copy number variant (CNV) is a segment of DNA in which differences of copy-number (number of copies of a DNA sequence or portions thereof) have been found by comparison of two or more genomes. The sequences and loci of the SNPs and CNVs known in the art so far, can be obtained from databases known to those skilled in the art. These databases are, e.g., The Database of Genomic Variants (DGV), the NCBI dbSNP database, the UCSC Genome Bioinformatics Site, the DatabasE of Chromosomal Imbalance and Phenotype in Humans using Ensembl Resources (DECIPHER), the HapMap Project, the Sanger Institute Copy Number Variation Project and the Human Structural Variation Project.

A similar assay may also be designed using two molecules that show a FRET effect instead of the fluorescence quenching. Here, one of the fluorophores is cleaved off upon a 5' to 3' nuclease activity of the DNA polymerase. Preferably, the DNA polymerase bears a 5' to 3' exonuclease activity, such as Taq polymerase. The spatial distance between the fluorophores is increased. The FRET efficiency decreases upon cleavage of fluorophore located at the 5' end. The fluorophore located at the 5' end may be the donor fluorophore or the acceptor fluorophore. Preferably, the fluorophore located at the 5' end may be the donor fluorophore. Preferably, this assay is conducted during a life-time PCR method. It may also be conducted quantitatively in a qPCR.

Determining the genotype may be performed by a hybridization- and/or PCR-based method as described herein. Preferably, determining the genotype may be performed in a PCR-based method. More preferably, determining the genotype may be performed in a PCR- and TaqMan®-based method.

A hybridization step with two or more probes may be performed. As used herein, the probes are preferably each labeled with one or more fluorophore(s), two different fluorophores or a fluorophore and a quencher. Further, the signal occurring from the probes is detected. Preferably, the FRET signal, the increase of fluorescence is detected. The probe may be a TaqMan® probe comprising a fluorophore and a quencher. Detection may be based on the presence or absence of fluorescence or on an increase or decrease of fluorescence.

The probes of the present invention may also be used for *in situ* hybridization, in particular in comparative *in situ* hybridization and/or fluorescent in situ hybridization (FISH). Herein, the probe may be fluorescently or radioactively labeled or may be detected by an antibody or an antibody derivative that is either labeled or that can be detected by a second antibody directed against its Fc part. Further, the probe may be conjugated with an enzyme that can generate a color from a precursor (e.g., a peroxidase, alkaline phosphatase).

Further, the probes of the present invention may be used in other methods based on probe hybridization. The probes of the present invention may be used in microarray methods. Preferably, the probes may be used in microarray validation. Moreover, the probes may be used in gene-knockdown quantification assays.

Further, the method of the present invention may be used for allele-specific PCR, wherein one of the probes may serve as a primer that is combined with a suitable primer or wherein two probes serve as forward and reverse primers. Preferably, one of the probes may serve as a primer that binds at the locus where the different alleles are located or are assumed to be located.

Further, the probe of the present invention may be used for microarray expression profiling, small RNA research, gene repair/exon skipping, splice variant detection, DNAzymes and/or comparative genome hybridization (GCH). These methods are well-known to those skilled in the art.

A probe of the present invention may also be used in a method for down-regulating expression of a gene. Therefore, the probe may serve as an antisense oligonucleotide such as RNAi (e.g., siRNA, miRNA, snRNA) as known in the art. The antisense oligonucleotide may interfere with mRNA of the target organism. The target organism may preferably be an animal, including human or a plant. Antisense oligonucleotides are well-known to those skilled in the art.

The composition of the present invention may be combined with a computer software for analyzing the data.

In a further aspect, the present invention relates to a library of at least two sets of probes, wherein the library comprises a plurality of sets of probes each of the probes having eight nucleotides with the general structure 5'-D - L - L - L - L - L - X - X-3' or 5'-D - L - L - L - L - X - X - X-3', wherein D is a DNA nucleotide, each L is a LNA nucleotide and each X is a LNA random nucleotide,
wherein within one set of probes all probes have identical nucleotide sequences with the exception of the two and/or three LNA random nucleotides, wherein at each position of a LNA random nucleotide the base is independently selected from adenine, cytosine, guanine and thymine and any possible sequence resulting from the base variation(s) at the two positions is represented by a probe in each set of probes; and
wherein one set of probes differs from another set of probes in the sequence of at least the DNA nucleotide D or an LNA nucleotide L.

As used herein, the term "library" refers to a collection of several sets of probes, wherein the DNA nucleotides and the LNA nucleotides may be located in the same position in each of the probes. Further, in a library of sets of probes, the random positions and the determined positions, and the discriminating position may be located at the same position in each probe of the library.

Said library may be designed to cover a plenty of targets in the genome of one species or the genome of different species. Ideally, such a probe library may be a library of probes for any technical problem.

For sets of probes of which three nucleotides are random nucleotides and five nucleotides are determined, 1024 sets of probes are sufficient to provide a suitable library of probes for any technical problem. As two sets of probes are needed of which at least the discriminating position of each one nucleotide is known, a library comprising 512 sets of probes is sufficient to apply to any technical problem.

For sets of probes of which two nucleotides are random nucleotides and six nucleotides are determined, 4096 sets of probes are sufficient to provide a suitable library of probes for any technical problem. As two sets of probes are needed of which at least the discriminating position of each one nucleotide is known, a library comprising 2048 sets of probes is sufficient to apply to any technical problem.

In a preferred embodiment, the library of sets of probes is characterized in that,
(i) number of sets amounts to at least 64, preferably 128, especially at least 256, particularly at least 512, more preferably at least 1024, most preferably 2048; and/or
(ii) the sets of probes are spatially separated from each other.

The term "spatially separated from each other" means that the probes may be stored separated from another, thus, e.g., in different vials or different wells. The vials may be plastic vials or glass vials, such as, e.g., a plastic cup, a screw cap vial or a sealed vial. A well may, e.g., refer to a well of a multiwell (multichamber plate, multititer plate) plate such as, e.g., an 8-well plate, 12-well plate, a 24-well plate, a 96-well plate, a 384-well plate or a 1536-well plate as known in the art. They may be stored at conditions suitable for single stranded DNA and/or LNA probes of the given length. They may be stored at room temperature, at 4°C, at -20°C, at -80°C or in liquid nitrogen. They may be stored in water, in an aqueous buffer, in an organic solvent, such as DMSO. Further, they may be freeze-dried.

A library of said probes may comprise at least two, at least 64, preferably 128, at least 256, at least 512, at least 1024, at least 2048, at least 4096 or more probes. The library of probes may comprise large parts of the genome of the organism or even the whole genome. The library of probes may comprise one, two, three, four, five or more different alleles for a particular locus of interest. There may be no, one, two, three or more loci of interest in a single gene.

The invention is further explained by the following examples and figures, which are intended to illustrate, but not limit the scope of the present invention.

### Figures

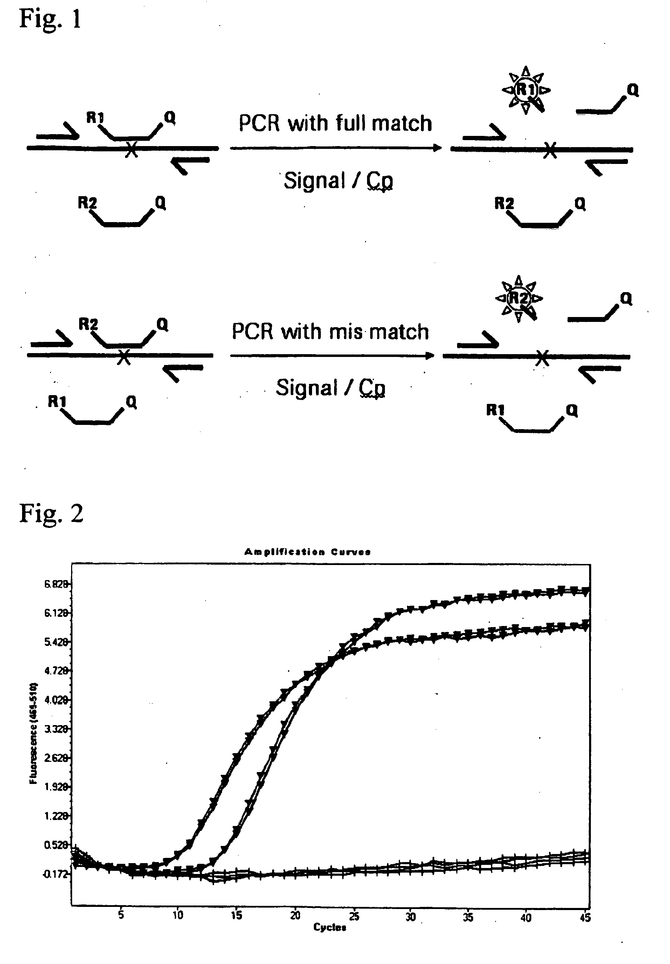

Fig. 1 shows a scheme of a PCR Dual Color Assay with two hydrolysis probes. The first probe (with reporter 1 referred to as R1) is hybridized and cleaved while presence of full match (to wild type sequence) in sample (top) allowing R1 to produce a signal, the second probe (with reporter 2 referred to as R2) is hybridized and cleaved while presence of full match (to mutant sequence) in sample (bottom) allowing R2 to produce a signal (Q = quencher).
Fig. 2 shows a mono color PCR assay with parameter 18S. The curves (triangle style) (top) show a positive Cp call and a sigmoidal amplification curve with full match of the probe at different sample concentrations. The curves (cross style) (bottom) refer to a negative Cp call and no sigmoidal amplification curve with mismatch of the probe at different sample concentrations.

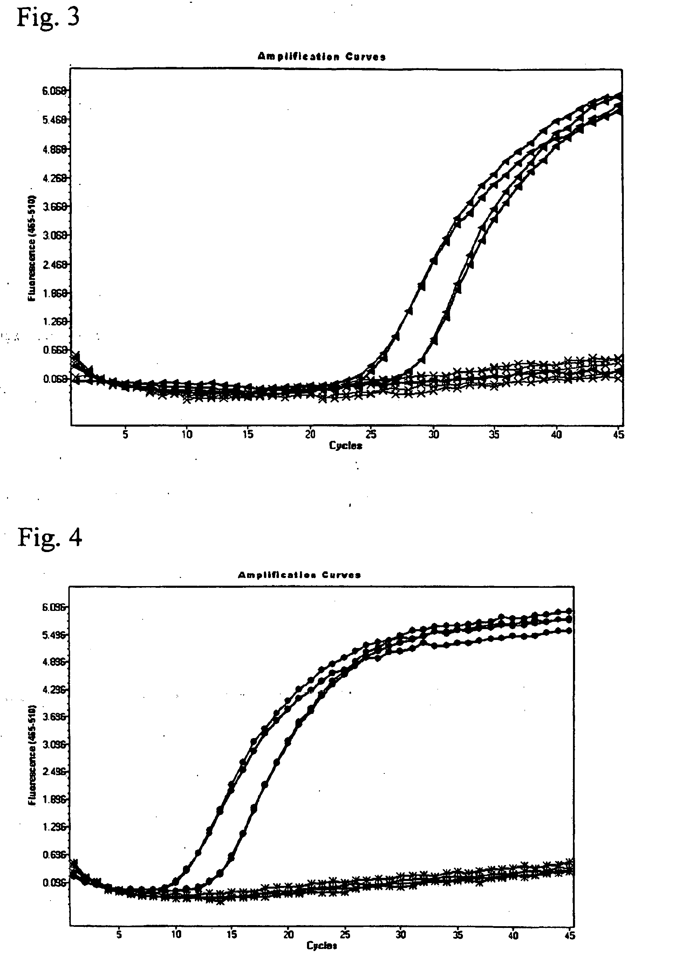

Fig. 3 shows a mono color PCR assay with parameter MNAT1. The curves (triangle style) (top) show a positive Cp call and a sigmoidal amplification curve with full match of the probe at different sample concentrations. The curves (cross style) (bottom) refer to a negative Cp call and no sigmoidal amplification curve with mismatch of the probe at different sample concentrations.
Fig. 4 shows a dual color PCR assay with parameter 18S. The curves (circle style) (top) show a positive Cp call and a sigmoidal amplification curve with full match of the probe at different sample concentrations. The curves (star style) (bottom) refer to a negative Cp call and no sigmoidal amplification curve with mismatch of the probe at different sample concentrations.

### Examples

### Example 1:

The experiment was carried out to show the discriminating power of the full match probe in comparison to the mis match probe, where the full match and the mismatch probe have the same nucleic acid sequence except one nucleic base in the middle of the probe sequence. The primer pairs are for both probes the same giving the same amplicon during PCR amplification. Because both probes have the same reporter dye the PCR experiment was performed in different wells in mono color mode only. To prove that the PCR performance is sufficient even with different sample concentrations the PCR was performed with two different dilutions of cDNA as target (in technical duplicates of each concentration). The target parameter in example 1 is 18S.

As expected the PCR with a probe having a full match sequence to the target sequence show a sigmoidal amplification curve and therefore a positive Cp call while the probe having a mis match to the target sequence show no sigmoidal amplification curve and a negative Cp call.

Experiment was carried out as illustrated in Fig. 1.
Mono Color PCR Assay with parameter 18S
General probe structure: 5'-D - L - L - L - L - L - X - X-3'
FAM full match probe sequence: 5'-X-t-T****A****T*T*G*-(AGCT)*(AGCT)*-Z-3'
FAM mismatch probe sequence: 5'-X-t-T****C****T*T*G*-(AGCT)*(AGCT)*-Z-3'
whereas X = FAM, t = dT
T*, A*, C* or G* = LNA T, A, C or G
(AGCT)* = wobble bases of LNA T, A, C and G (random position)
Z = BHQ2-Quencher (Black Hole Quencher 2)
Bold and italic fonts indicate discriminating position.
Primer forward sequence: gacggaccagagcgaaag (SEQ ID NO: 1)
Primer reverse sequence: cgtcttcgaacctccgact (SEQ ID NO: 2)
PCR Cycler: LightCycler® 480 Instrument, 384-well block (Roche Applied Science, Cat.
No.: 04 545 885 001)
PCR Reagents: LightCycler® 480 Probes Master (Roche Applied Science, Cat. No.: 04 707 494 001); LightCycler® 480 Multiwell Plate, 384 (Roche Applied Science, Cat. No.: 04 729 749 001)
Sample material: cDNA, reverse transcribed from RNA (Takara/Clontech, Cat. No.: 636538) with Transcriptor First Strand cDNA Synthesis Kit (Roche Applied Science, Cat. No.: 04 897 030 001). The cDNA synthesis step was performed as described in the pack insert of the corresponding kit.
Sample concentration: 50 ng and 5 ng per well in 10 µl final PCR volume
PCR protocol: see table below

### FAMKanal: 465:510

| | **°C** | **Time** | **RR(°C/s)(96)** | **AqMode** | **Cycles** |
|---|---|---|---|---|---|
| Preinkubation | 96 | 10min | 4.8(4,4) | - | **-** |
| Amplifikation denat | 95 | 10s | 4.8(4,4) | none | 30 |
| anneal. | 37 | 30s | 2.5(2,2) | none | - |
| elong | 50 | 30s | 4.8(4,4) | Single | - |
| Coding | 40 | 30s | 2.2(2.5) | none | - |

### Example 2

The experiment was carried out to show the discriminating power of the full match probe in comparison to the mis match probe, where the full match and the mis match probe have the same nucleic acid sequence except one nucleic base in the middle of the probe sequence. The primer pairs are for both probes the same giving the same amplicon during PCR amplification. Because both probes have the same reporter dye the PCR experiment was performed in different wells in mono color mode only. To prove that the PCR performance is sufficient even with different sample concentrations the PCR was performed with two different dilutions of cDNA as target (in technical duplicates of each concentration). The difference of example 2 compared to example 1 is a separate target parameter (parameter in example 2 is MNAT1).

As expected the PCR with a probe having a full match sequence to the target sequence show a sigmoidal amplification curve and therefore a positive Cp call while the probe having a mis match to the target sequence show no sigmoidal amplification curve and a negative Cp call.
Mono Color PCR Assay with parameter MNAT1
General probe structure: 5'-D - L - L - L - L - L - X - X-3'
FAM full match probe sequence: 5'-X-t-T****G****A*T*G*-(AGCT)*(AGCT)*-Z-3'
FAM mismatch probe sequence: 5'-X-t-T****A****A*T*G*-(AGCT)*(AGCT)*-Z-3'
whereas X = FAM, t = dT
T*,A*,C* or G*=LNA T,A,C or G
(AGCT)* = wobble bases of LNA T, A, C and G (random position)
Z = BHQ2-Quencher (Black Hole Quencher 2)
Bold and italic fonts indicate discriminating position.
Primer forward sequence: cccaaacctgtaaaaccagtg (SEQ ID NO: 3)
Primer reverse sequence: ttgtgaataggtgccagtgaa (SEQ ID NO: 4)
PCR Cycler: LightCycler® 480 Instrument, 384-well block (Roche Applied Science, Cat.
No.: 04 545 885 001)
PCR Reagents: LightCycler® 480 Probes Master (Roche Applied Science, Cat. No.: 04 707 494 001); LightCycler® 480 Multiwell Plate, 384 (Roche Applied Science, Cat. No.: 04 729 749 001)
Sample material: cDNA, reverse transcribed from RNA (Takara/Clontech, Cat. No.: 636538) with Transcriptor First Strand cDNA Synthesis Kit (Roche Applied Science, Cat. No.: 04 897 030 001). The cDNA synthesis step was performed as described in the pack insert of the corresponding kit.
Sample concentration: 50 ng and 5 ng per well in 10 µl final PCR volume
PCR protocol: see table below

### FAMKanal:465:510

| | °C | **Time** | **RR(°C/s)(96)** | **AqMode** | **Cycles** |
|---|---|---|---|---|---|
| Preinkubation | 95 | 10min | 4.8(4,4) | - | - |
| Amplification denat | 95 | 10s | 4.8(4,4) | none | 30 |
| anneal. | 37 | 30s | 2.5(2,2) | none | - |
| elong | 50 | 30s | 4.8(4,4) | Single | - |
| Coding | 40 | 30s | 2.2(2.5) | none | - |

### Example 3:

The experiment was carried out to show the discriminating power of the full match probe in comparison to the mis match probe, where the full match and the mis match probe have the same nucleic acid sequence except one nucleic base in the middle of the probe sequence. The primer pairs are for both probes the same giving the same amplicon during PCR amplification. Because in this example both probes have different reporter dyes the PCR experiment was performed in the same well in dual color mode. To prove that the PCR performance is sufficient even with different sample concentrations the PCR was performed with two different dilutions of cDNA as target (in technical duplicates of each concentration). The difference of example 3 compared to example 1 is the PCR mode mono color to dual color (the parameter in example 3 is 18S).

As expected the PCR with a probe having a full match sequence to the target sequence show a sigmoidal amplification curve in the corresponding fluorescence channel and therefore a positive Cp call while the probe having a mis match to the target sequence show no sigmoidal amplification curve in the corresponding fluorescence channel and a negative Cp call.

Dual Color PCR Assay with parameter 18S
General probe structures: 5'-D - L - L - L - L - L - X - X-3'
LC Yellow 555 full match probe sequence: 5'-Y-t-T*C*T*T*G*-(AGCT)*(AGCT)*-Z-3'
FAM mismatch probe sequence: 5'-X-t-T****A****T*T*G*-(AGCT)*(AGCT)*-Z-3'
whereas X = FAM, Y = LC Yellow 555 (LightCycler® Yellow 555), t = dT
T*, A*, C* or G* = LNA T, A, C or G
(AGCT)* = wobble bases of LNA T, A, C and G (random position)
Z = BHQ2-Quencher (Black Hole Quencher 2)
Bold and italic fonts indicate discriminating position.
Primer forward sequence: gacggaccagagcgaaag (SEQ ID NO: 1)
Primer reverse sequence: cgtcttcgaacctccgact (SEQ ID NO: 2)
PCR Cycler: LightCycler® 480 Instrument, 384-well block (Roche Applied Science, Cat.
No.: 04 545 885 001)
PCR Reagents: LightCycler® 480 Probes Master (Roche Applied Science, Cat. No.: 04 707 494 001); LightCycler® 480 Multiwell Plate, 384 (Roche Applied Science, Cat. No.: 04 729 749 001)
Sample material: cDNA, reverse transcribed from RNA (Takara/Clontech, Cat. No.: 636538) with Transcriptor First Strand cDNA Synthesis Kit (Roche Applied Science, Cat. No.: 04 897 030 001). The cDNA synthesis step was performed as described in the pack insert of the corresponding kit.
Sample concentration: 50 ng and 5 ng per well in 10 µl final PCR volume
PCR protocol: see table below

### FAMKanal: 465:510

| | **°C** | **Time** | **RR(°C/s)(96)** | **Aq Mode** | **Cycles** |
|---|---|---|---|---|---|
| Preinkubation | 95 | 10min | 4.8(4,4) | - | - |
| Amplifikation denat. | 95 | 10s | 4.8(4,4) | none | 30 |
| anneal. | 37 | 30s | 2.5(2,2) | none | - |
| elong | 50 | 30s | 4.8(4,4) | Single | - |
| Coding | 40 | 30s | 2.2(2.5) | none | - |

## Claims

1. A composition comprising a first set of probes and a second set of probes, each of the probes having eight nucleotides, wherein the eight nucleotides are composed of one to three DNA nucleotides and five to seven LNA (locked nucleic acid) nucleotides, wherein all probes of the first and the second set of probes have identical nucleotide sequences with the exception of
(i) the base(s) at one, two or three LNA random position(s); and
(ii) the base at a discriminating position,
wherein the one, two or three LNA random position(s) and the discriminating position are located at identical positions in all probes of the first and the second set; wherein at each LNA random position the base is independently selected from adenine, cytosine, guanine and thymine and any possible sequence resulting from the base variation(s) at the one, two or three LNA random position(s) is represented by at least one probe in each set of probes; and
wherein the base at the discriminating position is identical within each set of probes, but differs between the first and the second set of probes.

2. The composition of claim 1, wherein the discriminating position is at position 2, 3, 4, 5, 6 or 7 from the 5' end in each probe, preferably at position 3, 4, or 5, more preferably at position 4.

3. The composition of claim 1 or 2, wherein the nucleotide at position 1 from the 5' end is a DNA nucleotide.

4. The composition of any of claims 1 to 3, wherein each probe consists of
- one DNA and seven LNA nucleotides,
- two DNA and six LNA nucleotides, or
- three DNA and five LNA nucleotides.

5. The composition of any of claims 1 to 4,
a) wherein the set of probes has one LNA random position located at position 5; 6; 7; or 8 from the 5' end; or
b) wherein the set of probes has two LNA random positions located at positions 5 and 6; 5 and 7; 5 and 8; 6 and 7; 6 and 8; or 7 and 8 from the 5' end, preferably at positions 7 and 8; or
c) wherein the set of probes has three LNA random positions located at positions 5, 6 and 7; 5, 6 and 8; or 6, 7 and 8 from the 5' end, preferably at positions 6, 7 and 8.

6. The composition of any of claims 1 to 5, wherein the probes have the general structure 5'-D - L - L - L - L - X - X - X-3', wherein D is a DNA nucleotide, each L is a LNA nucleotide and each X is a LNA random position.

7. The composition of any of claims 1 to 5, wherein the probes have the general structure 5'-D - L - L - L - L - L - X - X-3', wherein D is a DNA nucleotide, each L is a LNA nucleotide and each X is a LNA random position.

8. The composition of any of claims 1 to 7, wherein the probes of first set of probes are labeled with a first marker and the probes of the second set of probes are labeled with a second marker, wherein the first marker is different from the second marker.

9. The composition of claim 8, wherein the first and the second marker are fluorescent markers.

10. The composition of claim 9, wherein the probes are hydrolysis probes additionally labeled with a quencher.

11. A method of determining the genotype at a locus of interest in a sample obtained from a subject, the method comprising
a) contacting the sample comprising genetic material with the composition of any of claims 1 to 10; and
b) detecting the binding of a probe of the first or the second set of probes to the genetic material, thereby determining the genotype at the locus.

12. The method of claim 11, wherein the locus is a single nucleotide.

13. The method of claim 11 or 12, wherein the method comprises
a) performing an amplifying step comprising contacting the sample with a set of primers to produce an amplification product including the locus of interest,
b) performing a hybridizing step comprising contacting the amplification product of step a) with the composition of any of claims 1 to 10; and
c) detecting the hybridizing of a probe of the first or the second set of probes to the genetic material, thereby determining the genotype at the locus.

14. The method of claims 11 to 13, wherein
(i) the detecting is by measuring presence or absence of fluorescence;
(ii) the detecting is in real-time;
(iii) the marker is selected from the group consisting of fluorescein, LC-Yellow 555, FAM, VIC, HEX, Rhodamine B, Rhodamine 6G, LC-Red 610, LC-Red 640, LC-Red 670, LC-Red 705, Cy3, Cy 3.5, Cy5, Cy5.5 and a quencher;
(iv) the amplifying step employs a polymerase enzyme having 5' to 3' exonuclease activity; and/or
(v) the sample is a biological sample, preferably a sample selected from the group consisting of a body fluid, a blood sample, a urine sample, serum, mucosa, sputum feces, epidermal sample, skin sample, cheek swab, sperm, amniotic fluid, cultured cells and bone marrow sample.

15. A library of at least two sets of probes, wherein the library comprises a plurality of sets of probes each of the probes having eight nucleotides with the general structure 5'-D-L-L-L-L-L-X-X-3' or 5'-D-L-L-L-L-X-X-X-3', wherein D is a DNA nucleotide, each L is a LNA nucleotide and each X is a LNA random nucleotide having,
wherein within one set of probes all probes have identical nucleotide sequences with the exception of the two and/or three LNA random nucleotides, wherein at each position of a LNA random nucleotide the base is independently selected from adenine, cytosine, guanine and thymine and any possible sequence resulting from the base variation(s) at the two positions is represented by a probe in each set of probes; and
wherein one set of probes differs from another set of probes in the sequence of at least the DNA nucleotide D or an LNA nucleotide L.

16. The library of sets of probes of claim 15,
(i) wherein number of sets amounts to at least 64, preferably 128, especially at least 256, particularly at least 512, more preferably at least 1024, most preferably 2048; and/or
(ii) wherein the sets of probes are spatially separated from each other.

## Patentansprüche

1. Zusammensetzung enthaltend einen ersten Sondensatz und einen zweiten Sondensatz, wobei jede Sonde acht Nukleotide aufweist, wobei die acht Nukleotide aus ein bis drei DNA-Nukleotiden und fünf bis sieben LNA-(gesperrter Nukleinsäure; locked nucleic acid)-Nucleotiden zusammengesetzt sind, wobei alle Sonden des ersten und des zweiten Sondensatzes identische Nukleotidsequenzen aufweisen, mit der Ausnahme
(i) der Base(n) an eins, zwei oder drei zufälligen LNA-Position(en); und
(ii) Der Base an der Unterscheidungsposition,
wobei die eins, zwei oder drei zufälligen LNA-Position(en) und die Unterscheidungsposition in allen Proben des ersten und des zweiten Satzes an identischen Positionen lokalisiert sind;
wobei an jeder zufälligen LNA-Position die Base unabhängig voneinander ausgewählt ist aus Adenin, Cytosin, Guanin und Thymin und jede mögliche Sequenz, die aus der/den Basenvariation(en) an der einen, zwei oder drei zufälligen LNA-Position(en) resultiert, durch mindestens eine Sonde in jedem Satz der Sonden repräsentiert ist; und
wobei die Base an der Unterscheidungsposition innerhalb eines jeden Sondensatzes identisch ist, aber sich bei dem ersten und dem zweiten Sondensatz voneinander unterscheidet.

2. Zusammensetzung nach Anspruch 1, wobei die Unterscheidungsposition an Position 2, 3, 4, 5, 6 oder 7 vom 5'-Ende jeder Sonde, vorzugsweise an Position 3, 4 oder 5, weiter bevorzugt an Position 4, ist.

3. Zusammensetzung nach Anspruch 1 oder 2, wobei das Nukleotid an Position 1 vom 5'-Ende ein DNA-Nukleotid ist.

4. Zusammensetzung nach einem beliebigen der Ansprüche 1 bis 3, wobei jede Sonde besteht aus
- einem DNA- und 7 LNA-Nukleotiden,
- zwei DNA- und 6 LNA-Nukleotiden, oder
- drei DNA- und 5 LNA-Nukleotiden.

5. Zusammensetzung nach einem beliebigen der Ansprüche 1 bis 4,
a) wobei der Probensatz genau eine zufällige LNA-Position aufweist, die an Position 5; 6; 7; oder 8 vom 5'-Ende lokalisiert ist;
b) wobei der Probensatz genau zwei zufällige LNA-Positionen aufweist, die an Positionen 5 und 6; 5 und 7; 5 und 8; 6 und 7; 6 und 8; oder 7 und 8 vom 5'-Ende lokalisiert sind, bevorzugt an Positionen 7 und 8; oder
c) wobei der Probensatz genau drei zufällige LNA-Positionen aufweist, die an Positionen 5, 6 und 7; 5, 6 und 8; oder 6, 7 und 8 vom 5'-Ende lokalisiert sind, bevorzugt an Positionen 6, 7 und8.

6. Zusammensetzung nach einem beliebigen der Ansprüche 1 bis 5, wobei die Sonden die allgemeine Formel 5'-D-L-L-L-L-X-X-X-3' aufweisen, wobei D ein DNA-Nukleotid ist, jedes L ein LNA-Nukleotid ist und jedes X eine zufällige LNA-Position ist.

7. Zusammensetzung nach einem beliebigen der Ansprüche 1 bis 5, wobei die Sonden die allgemeine Formel 5'-D-L-L-L-L-L-X-X-3' aufweisen, wobei D ein DNA-Nukleotid ist, jedes L ein LNA-Nukleotid ist und jedes X eine zufällige LNA-Position ist.

8. Zusammensetzung nach einem beliebigen der Ansprüche 1 bis 7, wobei die Sonden des ersten Sondensatzes mit einem ersten Marker markiert sind und die Sonden des zweiten Sondensatzes mit einem zweiten Marker markiert sind, wobei der erste Marker sich von dem zweiten Marker unterscheidet.

9. Zusammensetzung nach Anspruch 8, wobei der erste und der zweite Marker Fluoreszenz-Marker sind.

10. Zusammensetzung nach Anspruch 9, wobei die Sonden Hydrolyse-Sonden sind, die zusätzlich mit einem Quencher markiert sind.

11. Verfahren zum Bestimmen des Genotyps an einem Lokus von Interesse in einer Probe, die von einem Subjekt erhalten wurde, wobei das Verfahren umfasst:
a) Inkontaktbringen der Probe, die genetisches Material enthält, mit der Zusammensetzung nach einem beliebigen der Ansprüche 1 bis 10; und
b) Nachweisen der Bindung einer Sonde des ersten oder zweiten Sondensatzes an das genetische Material, wodurch der Genotyp an dem Lokus bestimmt wird.

12. Verfahren nach Anspruch 11, wobei der Lokus ein einzelnes Nukleotid ist.

13. Verfahren nach Anspruch 11 oder 12, wobei das Verfahren umfasst:
a) Durchführen eines Amplifizierungsschritts umfassend das Inkontaktbringen der Probe mit einem Primer-Satz um ein Amplifikationsprodukt, welches den Lokus von Interesse einschießt, herzustellen,
b) Durchführen eines Hybridisierungsschritts umfassend das Inkontaktbringen des Amplifikationsprodukts von Schritt a) mit der Zusammensetzung nach einem beliebigen der Ansprüche 1 bis 10; und
c) Nachweisen der Hybridisierung einer Sonde des ersten oder zweiten Sondensatzes an das genetische Material, wodurch der Genotyp an dem Lokus bestimmt wird.

14. Verfahren nach den Ansprüchen 11 bis 13, wobei
(i) das Nachweisen durch Messen der Gegenwart oder Abwesenheit von Fluoreszenz erfolgt;
(ii) das Nachweisen in Echtzeit erfolgt;
(iii) Der Marker ausgewählt ist aus der Gruppe bestehend aus Fluorescein, LC-Yellow 555, FAM, VIC, HEX, Rhodamin B, Rhodamin 6G, LC-Red 610, LC-Red 640, LC-Red 670, LC-Red 705, Cy3, Cy3.5, Cy5, Cy5.5 und mit einem Quencher;
(iv) der Amplifizierungsschritt ein Polymerase-Enzym mit 5' nach 3' Exonuklease-Aktivität einsetzt; und/oder
(v) die Probe eine biologische Probe ist, vorzugsweise eine Probe ausgewählt aus der Gruppe bestehend aus einer Körperflüssigkeit, einer Blutprobe, einer Urinprobe, Serum, Mukosa, Spucke, Fezes, einer epidermischen Probe, einer Hautprobe, einem Wangenabstrich, Sperma, Fruchtwasser, kultivierten Zellen und einer Knochenmarksprobe.

15. Bibliothek von mindestens zwei Sondensätzen, wobei die Bibliothek eine Vielzahl von Sondensätzen umfasst, wobei jede der Sonden 8 Nukleotide mit der allgemeinen Formel 5'-D-L-L-L-L-X-X-X-3' oder 5'-D-L-L-L-L-L-X-X-3' aufweist, wobei D ein DNA-Nukleotid ist, jedes L ein LNA-Nukleotid ist und jedes X ein zufälliges LNA-Nukleotid ist,
wobei innerhalb eines Sondensatzes alle Sonden identische Nukleotidsequenzen aufweisen mit der Ausnahme von zwei und/oder drei zufälligen LNA-Nukleotiden, wobei an jeder Position eines zufälligen LNA-Nukleotids die Base unabhängig voneinander ausgewählt ist aus Adenin, Cytosin, Guanin und Thymin und jede mögliche Position, die aus der/den Basenvariation/en an den beiden Positionen resultiert, durch eine Sonde in jedem Sondensatz repräsentiert ist; und
wobei ein Sondensatz sich von dem anderen Sondensatz in der Sequenz mindestens des DNA-Nukleotids D oder eines LNA-Nukleotids L unterscheidet.

16. Bibliothek von Sondensätzen nach Anspruch 15,
(i) wobei die Anzahl der Sätze mindestens 64, vorzugsweise 128, insbesondere mindestens 256, besonders mindestens 512, mehr besonders mindestens 1024, am meisten bevorzugt 2048 beträgt; und/oder
(ii) wobei die Sondensätze räumlich voneinander getrennt sind.

## Revendications

1. Composition comprenant un premier jeu de sondes et un deuxième jeu de sondes, chacune des sondes contenant huit nucléotides, les huit nucléotides étant composés de un à trois nucléotides d'ADN et de cinq à sept nucléotides LNA (acide nucléique bloqué), dans laquelle toutes les sondes du premier et du deuxième jeu de sondes possèdent des séquences nucléotidiques identiques à l'exception de
(i) la/les bases à une, deux ou trois positions aléatoires des LNA ; et
(ii) la base à une position de discrimination ;
dans laquelle les une, deux ou trois positions aléatoires des LNA et la position de discrimination sont situées à des positions identiques dans toutes les sondes du premier et du deuxième jeu ;
dans laquelle à chaque position aléatoire des LNA, la base est choisie de manière indépendante parmi l'adénine, la cytosine, la guanine et la thymine, et n'importe quelle séquence possible qui résulte de la/des variations de bases aux une, deux ou trois positions aléatoires des LNA est représentée par au moins une sonde dans chaque jeu de sondes ; et dans laquelle la base à la position de discrimination est identique au sein de chaque jeu de sondes, mais diffère entre le premier et le deuxième jeu de sondes.

2. Composition selon la revendication 1, dans laquelle la position de discrimination est située à la position 2, 3, 4, 5, 6 ou 7 à partir de l'extrémité 5' dans chaque sonde, de préférence à la position 3, 4 ou 5, de manière plus préférée à la position 4.

3. Composition selon la revendication 1 ou 2, dans laquelle le nucléotide à la position 1 à partir de l'extrémité 5' est un nucléotide d'ADN.

4. Composition selon l'une quelconque des revendications 1 à 3, dans laquelle chaque sonde est constituée par :
- un nucléotide d'ADN et sept nucléotides de LNA ;
- deux nucléotides ADN et six nucléotides de LNA ; ou
- trois nucléotides ADN et cinq nucléotides de LNA.

5. Composition selon l'une quelconque des revendications 1 à 4,
a) dans laquelle le jeu de sondes possède une position aléatoire de LNA située à la position 5, 6, 7 ou 8 à partir de l'extrémité 5' ; ou
b) dans laquelle le jeu de sondes possède deux positions aléatoires de LNA situées aux positions 5 et 6 ; 5 et 7 ; 5 et 8 ; 6 et 7 ; 6 et 8 ; ou 7 et 8 à partir de l'extrémité 5', de préférence aux positions 7 et 8 ; ou
c) dans laquelle le jeu de sondes possède trois positions aléatoires de LNA situées aux positions 5, 6 et 7 ; 5, 6 et 8 ; ou 6, 7 et 8 à partir de l'extrémité 5', de préférence aux positions 6, 7 et 8.

6. Composition selon l'une quelconque des revendications 1 à 5, dans laquelle les sondes possèdent la structure générale 5'-D-L-L-L-L-X-X-X-3', dans laquelle D représente un nucléotide d'ADN, chaque L représente un nucléotide de LNA et X représente une position aléatoire de LNA.

7. Composition selon l'une quelconque des revendications 1 à 5, dans laquelle les sondes possèdent la structure générale 5'-D-L-L-L-L-L-X-X-3', dans laquelle D représente un nucléotide d'ADN, chaque L représente un nucléotide de LNA et X représente une position aléatoire de LNA.

8. Composition selon l'une quelconque des revendications 1 à 7, dans laquelle les sondes du premier jeu de sondes sont marquées avec un premier marqueur et les sondes du deuxième jeu de sondes sont marquées avec un deuxième marqueur, le premier marqueur étant différent du deuxième marqueur.

9. Composition selon la revendication 8, dans laquelle le premier et le deuxième marqueur sont des marqueurs fluorescents.

10. Composition selon la revendication 9, dans laquelle les sondes sont des sondes d'hydrolyse marquées en outre avec un extincteur de luminescence.

11. Procédé de détermination du génotype à un site actif d'intérêt dans un échantillon que l'on obtient à partir d'un sujet, le procédé comprenant :
a) la mise en contact de l'échantillon comprenant un matériau génétique avec la composition selon l'une quelconque des revendications 1 à 10 ; et
b) la détection de la liaison d'une sonde du premier ou du deuxième jeu de sondes au matériau génétique, pour ainsi déterminer le génotype au site actif.

12. Procédé selon la revendication 11, dans lequel le site actif est un nucléotide unique.

13. Procédé selon la revendication 11 ou 12, dans lequel le procédé comprend :
a) la mise en oeuvre d'une étape d'amplification comprenant la mise en contact de l'échantillon avec un jeu d'amorces pour obtenir un produit d'amplification englobant le site actif d'intérêt ;
b) la mise en oeuvre d'une étape d'hybridation comprenant la mise en contact du produit d'amplification de l'étape a) avec la composition selon l'une quelconque des revendications 1 à 10 ; et
c) la détection de l'hybridation d'une sonde du premier ou du deuxième jeu de sondes au matériau génétique, pour ainsi déterminer le génotype au site actif.

14. Procédé selon les revendications 11 à 13, dans lequel
(i) la détection s'effectue en mesurant la présence ou l'absence de fluorescence ;
(ii) la détection a lieu en temps réel ;
(iii) le marqueur est sélectionné parmi le groupe constitué par la fluorescéine, LC-Yellow 555, FAM, VIC, HEX, la rhodamine B, la rhodamine 6G, LC-Red 610, LC-Red 640, LC-Red 670, LC-Red 705, Cy3, Cy3.5, Cy5, Cy5.5 et un extincteur de la fluorescence ;
(iv) l'étape d'amplification utilise une enzyme polymérase possédant une activité d'exonucléase de 5' à 3' ; et/ou
(v) l'échantillon est un échantillon biologique, de préférence un échantillon choisi parmi le groupe constitué par un liquide organique, un échantillon sanguin, un échantillon d'urine, du sérum, de la muqueuse, du crachat, des matières fécales, un échantillon épidermique, un échantillon cutané, un frottis de la paroi buccale, du sperme, du fluide amniotique, des cellules cultivées et un échantillon de moelle osseuse.

15. Banque d'au moins deux jeux de sondes, la banque comprenant plusieurs jeux de sondes, chacune des sondes possédant huit nucléotides possédant la structure générale 5'-D-L-L-L-L-L-X-X-3' ou 5'-D-L-L-L-L-X-X-X-3' dans laquelle D représente un nucléotide d'ADN, chaque L représente un nucléotide de LNA et X représente une position aléatoire de LNA ;
dans laquelle, au sein d'un jeu de sondes, toutes les sondes possèdent des séquences nucléotidiques identiques à l'exception des deux et/ou trois nucléotides aléatoires de LNA, dans laquelle, à chaque position d'un nucléotide aléatoire de LNA, la base est choisie de manière indépendante parmi l'adénine, la cytosine, la guanine et la thymine, et n'importe quelle séquence possible résultant de la/des variations de bases aux deux positions est représentée par une sonde dans chaque jeu de sondes ; et
dans laquelle un jeu de sondes diffère d'un autre jeu de sondes dans la séquence d'au moins le nucléotide D d'ADN ou d'un nucléotide L de LNA.

16. Banque de jeux de sondes selon la revendication 15,
(i) dans laquelle le nombre de jeux s'élève à au moins 64, de préférence à 128, de manière spécifique à au moins 256, en particulier à au moins 512, de manière plus préférée à au moins 1024, de manière de loin préférée à 2048 ; et/ou
(ii) dans laquelle les jeux de sondes sont séparés les uns des autres dans l'espace.
